# EUROPEAN PATENT SPECIFICATION

(11) **EP 3 049 815 B1**
(45) Date of publication and mention of the grant of the patent: **13.03.2019**
(21) Application number: 14776859.2
(22) Date of filing: 26.09.2014
(51) Int. Cl.: G01N 33/74, A61K 39/00

(54) **GOODPASTURE ANTIGEN BINDING PROTEIN DETECTION AND INHIBITION AND ITS USE IN DIABETES**
NACHWEIS UND HEMMUNG DES GOODPASTURE-ANTIGEN-BINDENDEN PROTEINS UND DESSEN VERWENDUNG BEI DIABETES
DÉTECTION ET INHIBITION DE LA PROTÉINE DE LIAISON À L'ANTIGÈNE DE GOODPASTURE ET SON UTILISATION DANS LA CADRE DU DIABÈTE

(30) Priority: 27.09.2013 US 201361883792 P
(43) Date of publication of application: 03.08.2016
(73) Proprietor: Fibrostatin, Sociedad Limitada, 46005 Valencia (ES)
(72) Inventor: SAUS, Juan, E-46005 Valencia (ES); REVERT, Fernando, E-46005 Valencia (ES); REVERT-ROS, Francisco, E-46005 Valencia (ES)
(74) Representative: Zwicker, Jörk
(86) International application number: PCT/EP2014/070629
(87) International publication number: WO 2015/044352

(56) References cited:
- US-A1- 2010 021 935
- US-A1- 2010 021 935
- US-A1- 2011 105 545
- US-A1- 2011 105 545
- GUO JUN ET AL: "Palmitate-Induced Inhibition of Insulin Gene Expression in Rat Islet beta-Cells Involves the Ceramide Transport Protein", CELLULAR PHYSIOLOGY AND BIOCHEMISTRY, vol. 26, no. 4-5, 2010, pages 717-728, XP002734254, ISSN: 1015-8987
- SEHAMUDDIN GALADARI ET AL: "Role of ceramide in diabetes mellitus: evidence and mechanisms", LIPIDS IN HEALTH AND DISEASE, vol. 12, no. 1, 98, 8 July 2013 (2013-07-08), pages 1-16, XP021156761, BIOMED CENTRAL, LONDON, GB ISSN: 1476-511X, DOI: 10.1186/1476-511X-12-98
- CHIARA MENCARELLI ET AL: "The ceramide transporter and the Goodpasture antigen binding protein: one protein - one function?", JOURNAL OF NEUROCHEMISTRY, vol. 113, no. 6, 1 April 2010 (2010-04-01) , pages 1369-1386, XP055160200, ISSN: 0022-3042, DOI: 10.1111/j.1471-4159.2010.06673.x
- GUO J ET AL: "Palmitate-induced inhibition of insulin gene expression in rat islet [beta]-cells involves the ceramide transport protein", CELLULAR PHYSIOLOGY AND BIOCHEMISTRY, KARGER, BASEL, CH; BIOSCIENCES INFORMATION SERVICE, PHILADELPHIA, PA, US, vol. 26, no. 4-5, 1 January 2010 (2010-01-01), pages 17-28, XP002734254, ISSN: 1015-8987, DOI: 10.1159/000322339 [retrieved on 2010-10-29]
- SEHAMUDDIN GALADARI ET AL: "Role of ceramide in diabetes mellitus: evidence and mechanisms", LIPIDS IN HEALTH AND DISEASE, BIOMED CENTRAL, LONDON, GB, vol. 12, no. 1, 8 July 2013 (2013-07-08), page 98, XP021156761, ISSN: 1476-511X, DOI: 10.1186/1476-511X-12-98
- Chiara Mencarelli ET AL: "The ceramide transporter and the Goodpasture antigen binding protein: one protein - one function?", Journal of Neurochemistry, vol. 113, no. 6, 1 April 2010 (2010-04-01) , pages 1369-86, XP055160200, ISSN: 0022-3042, DOI: 10.1111/j.1471-4159.2010.06673.x

## Description

### Background of the Invention

The conformation of the non-collagenous (NC1) domain of the α3 chain of the basement membrane collagen IV (α3NC1) depends in part on phosphorylation. Goodpasture Antigen Binding Protein (GPBP) (WO 00/50607; WO 02/061430) is a novel non-conventional protein kinase that catalyzes the conformational isomerization of the α3NC1 domain during its supramolecular assembly, resulting in the production and stabilization of multiple α3NC1 conformers in basement membranes. Elevated levels of GPBP have been associated with the production of non-tolerized α3NC1 conformers, which conduct the autoimmune response mediating Goodpasture (GP) disease. In GP patients, autoantibodies against the α3NC1 (also known as GP antigen) cause a rapidly progressive glomerulonephritis and often lung hemorrhage, the two cardinal clinical manifestations of the GP disease.

*COL4A3BP* is a gene for regulation of structural protein supramolecular organization inside (i.e. myosin) and outside (i.e. collagen IV) of the cell. The primary gene product GPBP (also known as GPBP-1 or 77 kD GPBP) resides mainly in the extracellular compartment where it can be found circulating or bound to collagen IV. Interestingly, the gene also expresses alternative isoforms which remain mainly intracellular and soluble in the cytosol (i.e. GPBP-2 also known as GPBPΔ26 or CERT) or insoluble and associated to cellular membranes and organelles (i.e. GPBP-3 also known as 91 kD GPBP) (WO 00/50607; WO 2010/009856; Revert-Ros et al., 2011, J Biol. Chem 286, 35030-35043).

Guo and coworkers (Guo J. et al., Cell. Physiol. Biochem. 2010; 26:717-728) studied the role of endogenous ceramide in the palmitate impairment of proinsulin gene expression in pancreatic islet β-cells. They showed that suppression of ceramide transport protein (CERT) mRNA with small interfering mRNA contributed to intracellular ceramide accumulation in response to chronic palmitate exposure and impairment of proinsulin gene expression. They concluded from their experiments that palmitate-mediated dysfunction of ceramide transport may contribute to intracellular ceramide accumulation and result in dysfunction of pancreatic β-cells by affecting binding of transcription factors to the insulin promoter.

### Summary of the invention

In one aspect, the present invention provides a GPBP-1 inhibitor, including but not limited to an anti-GPBP-1 antibody, for use in the treatment of the pre-diabetic state.

In a further aspect, the present invention provides methods for diagnosing a pre-diabetic state comprising
(a) determining an amount of GPBP-1 in a sample from a subject at risk of being in a pre-diabetic state; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample indicates the presence of a pre-diabetic state in the subject.

In another aspect, the present invention provides methods for diagnosing a propensity to develop T2D, comprising
(a) determining an amount of GPBP-1 in a sample from a subject at risk of having a pre-diabetic state; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample indicates a propensity to develop T2D in the subject.

### Detailed Description of the Invention

Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

As used herein, the singular forms "a", "an" and "the" include plural referents unless the context clearly dictates otherwise. "And" as used herein is interchangeably used with "or" unless expressly stated otherwise.

Unless clearly indicated otherwise by the context, embodiments disclosed for one aspect of the invention can be used in other aspects of the invention as well, and in combination with embodiments disclosed in other aspects of the invention.

In a further aspect, the invention provides a GPBP-1 inhibitor for use in the treatment of the pre-diabetic state. In this aspect, the subject is one who has a pre-diabetic state. As used herein, a "pre-diabetic state" is the state in which some but not all of the diagnostic criteria for diabetes are met. Thus, the pre-diabetic state may comprise: 1) having impaired fasting glucose tolerance, which is a condition whereby the response of beta cells to an oral glucose challenge (OGT) is deficient or 2) having consistently elevated fasting glucose (IFG), which is a condition in which the fasting blood glucose is elevated above what is considered normal levels but is not high enough to be classified as diabetes mellitus. The pre-diabetic state may be associated with insulin resistance and increased risk of cardiovascular pathology. Individuals with a pre-diabetic state are at a relatively high risk of developing T2D.

As used herein, "GPBP" will refer to any GPBP isoform, i.e.: GPBP-1 (SEQ ID NO:10), GPBP-2 (SEQ ID NO:11), and GPBP-3 (SEQ ID NO:12). GPBP-1 (also known as 77 kD GPBP) resides mainly in the extracellular compartment where it can be found circulating or bound to collagen IV. GPBP-2 (also known as GPBPΔ26 or CERT) is a GPBP-1 variant of 598-residue-long generated by mRNA alternative exon splicing that remains mainly intracellular and cytosolic. GPBP-3 (also known as 91 kD GPBP) is a GPBP-1 variant of 707-residue-long arising from alternative non-canonical mRNA translation initiation that remains mainly intracellular and associated with cellular membranes including the outer side of the plasma membrane and organelles.

As used herein a "GPBP inhibitor" will refer to any compound or molecule that reduces the activity or the expression of all or individual GPBP isoforms.

As demonstrated by the inventors in the examples that follow, GPBP-1 mediates pathogenesis in an animal model of T2D, and inhibition of GPBP-1 activity delayed or precluded hyperglycemia. Thus, inhibitors of GPBP-1 expression or activity can be used to treat those with a pre-diabetic state. The inventors have recently discovered (data not shown) that GPBP-1 performs at least three key activities related through a cycle: protein kinase (cytoplasm and, eventually, the nucleus and the endoplasmic reticulum); chaperone (endoplasmic reticulum) and inflammatory "cytokine" (extracellular compartment). The cycle's progression toward the cell exterior is regulated in part by GPBP-3 expression (WO 2010/009856) and is completed because the extracellular GPBP-1 is recaptured and returns to the cytoplasm. The evidence also shown that GPBP-2 expression can compensate GPBP-1 deficiency *in vivo* (Revert-Ros et al., 2011, J Biol Chem 286, 35030-35043) supporting that GPBP-2 expression is somehow contributing to the normal progression of the GPBP cycle. The existence of the GPBP cycle with intra- and extracellular stages means that a disorder caused by exaggerated activity in the cytosol can be directly interfered with by an inhibitor compound, but also indirectly by either an inhibitor of the activity in the ER or an antibody that blocks extracellular activity; and vice versa, a disorder caused by exaggerated extracellular activity can be directly interfered with by a blocking antibody and indirectly by an inhibitor of intracellular activity. Thus, in light of the GPBP cycle inhibitors of any isoform of GPBP (GPBP-1, GPBP-2, and/or GPBP-3) can be used to treat those with a pre-diabetic state.

In all aspects and embodiments, the subject may be any mammal, including but not limited to a human, dog, cat, horse, or livestock (cow, sheep, etc.). In a most preferred embodiment, the subject is a human subject.

As used herein, "treat" or "treating" means accomplishing one or more of the following: (a) reducing the severity of the disorder; (b) limiting or preventing development of symptoms or complications characteristic of the disorder(s) being treated; (c) inhibiting worsening of symptoms or complications characteristic of the disorder(s) being treated; (d) limiting or preventing recurrence of symptoms or complications in patients that were previously symptomatic for the disorder(s).

Symptoms and complications of T2D that can be limited with the methods of the invention include, but are not limited to hyperglycemia, insulin resistance, diabetic nephropathy, diabetic neuropathy, diabetic retinopathy, proteinuria, impaired glomerular clearance, diabetic circulatory disorders, and kidney failure. Any amount of limiting of these symptoms/complications is of great benefit to a subject with T2D.

The invention can be used to treat a subject with pre-diabetes by, for example, limiting/slowing progression of hyperglycemia, insulin resistance, and/or cardiovascular pathologies in the subject and/or limiting/slowing progression of the subject to T2D.

In one embodiment, the subject has increased circulating GPBP-1 relative to control. This embodiment may help identify subjects that may most benefit from treatment. Such "increase" can be any amount of increase in GPBP-1 relative to control (such as control sample from normal subject, or previously determined "normal" levels of GPBP-1 in a control population), for example, 5%, 10%, 15%, 20%, 25%, 50%, 75%, 100%, or greater. In one embodiment, a normal value of GPBP-1 as a reference for a standard curve is less than 10 ng/ml in plasma. In various embodiments, a normal GPBP range may be between ∼ 1 ng/ml-10 ng/ml in plasma. Thus, in one embodiment, subjects identified as having more than 10 ng/ml of GPBP-1 in their plasma are treated according to the invention. Methods for determining the amount of circulating GPBP are known (see WO 2010/009856 and US 7935492).

Any suitable GPBP-1 inhibitor may be used in the invention. In one embodiment, the GPBP-1 inhibitor comprises an anti-GPBP-1 antibody, such as a monoclonal or polyclonal antibody. As used herein, "anti-GPBP antibody" means that the antibodies bind to all or individual GPBP isoforms. In a preferred embodiment that can be combined with any other embodiment, the antibody is a monoclonal antibody, such as a humanized monoclonal antibody. The term antibody as used herein is intended to include antibody fragments thereof which are selectively reactive with the polypeptides of the invention, or fragments thereof. Antibodies can be fragmented using conventional techniques or synthesized through genetic engineering using recombinant DNA, and the fragments screened for utility in the same manner as described above for whole antibodies. For example, F(ab')2 fragments can be generated by treating antibody with pepsin. The resulting F(ab')2 fragment can be treated with papain to produce Fab fragments. Examples of monoclonal antibody fragments include (i) a Fab fragment, a monovalent fragment consisting essentially of the VL, VH, CL and CH1 domains; (ii) F(ab)2 and F(ab')2 fragments, bivalent fragments comprising two Fab fragments linked by a disulfide bridge at the hinge region; (iii) a Fd fragment consisting essentially of the VH and CH1 domains; (iv) a Fv fragment consisting essentially of the VL and VH domains of a single arm of an antibody, such as scFV, tandem di-scFV, diabodies, tri(a)bodies, etc. (v) a dAb fragment (Ward et al., (1989) Nature 341:544-546), which consists essentially of a VH domain; and (vi) one or more isolated CDRs or a functional paratope.

In various further embodiments, the GPBP-1 inhibitor comprises one or more inhibitors selected from the group consisting of:
(a) an aptamer selective for GPBP-1;
(c) an inhibitory nucleic acid selective for GPBP-1 mRNA;
(d) a peptide comprising an amino acid sequence of general formula X1-SHCIX2-X3 (SEQ ID NO: 1)
   wherein X1 is 0-10 amino acids of the sequence ATTAGILATL (SEQ ID NO: 2);
   X2 is E or Q; and
   X3 is 0-10 amino acids of the sequence LMVKREDSWQ (SEQ ID NO: 3);
(e) a peptide comprising an amino acid sequence selected from the group consisting of SHCIE (SEQ ID NO: 4), SHCIQ (SEQ ID NO: 5), ILATLSHCIELMVKR (SEQ ID NO: 6), ILATLSHCIQLMVKR (SEQ ID NO: 7), and LATLSHCIELMVKR (SEQ ID NO: 8); and
(f) I-20:

Sequences of the polypeptides recited in the above are known in the art; see, for example, US 7,326.768; 7,935.492; 6,579.969; and 7,147.855.

Synthesis of the other GPBP inhibitors disclosed herein can be carried out by those of skill in the art, based on the teachings in WO2011/054530.

The peptides can further be derivatized to provide enhanced half-life, such as by the addition of polyethylene glycol (PEG) or as otherwise known in the art. The peptides may comprise L-amino acids, D-amino acids (which are resistant to L-amino acid-specific proteases *in vivo),* a combination of D- and L-amino acids, and various "designer" amino acids (e.g., β-methyl amino acids, Cα-methyl amino acids, and Nα-methyl amino acids, etc.) to convey special properties. Synthetic amino acids include ornithine for lysine, and norleucine for leucine or isoleucine.

In addition, the peptides can have peptidomimetic bonds, such as ester bonds, to prepare peptides with novel properties. For example, a peptide may be generated that incorporates a reduced peptide bond, i.e., R₁-CH₂-NH-R₂, where R₁ and R₂ are amino acid residues or sequences. A reduced peptide bond may be introduced as a dipeptide subunit. Such a peptide would be resistant to protease activity, and would possess an extended half-live *in vivo.*

The term "peptide" is used in its broadest sense to refer to a sequence of subunit amino acids, amino acid analogs, or peptidomimetics. The subunits are linked by peptide bonds, although the peptide can comprise further moieties that are not necessarily linked to the peptide by a peptide bond. For example, as discussed above, the peptide can further comprise a non-amino acid molecule that contains an aromatic ring.

The peptides described herein may be chemically synthesized or recombinantly expressed. Recombinant expression can be accomplished using standard methods in the art, as disclosed above. Such expression vectors can comprise bacterial or viral expression vectors, and such host cells can be prokaryotic or eukaryotic.

The peptide/antibody inhibitors may be administered together in a pharmaceutical composition with a pharmaceutically acceptable carrier. The pharmaceutical composition may comprise in addition to the peptide/antibody inhibitor (a) a lyoprotectant; (b) a surfactant; (c) a bulking agent; (d) a tonicity adjusting agent; (e) a stabilizer; (f) a preservative and/or (g) a buffer.

In some embodiments, the buffer in the pharmaceutical composition is a Tris buffer, a histidine buffer, a phosphate buffer, a citrate buffer or an acetate buffer. The pharmaceutical composition may also include a lyoprotectant, e.g. sucrose, sorbitol or trehalose. In certain embodiments, the pharmaceutical composition includes a preservative e.g. benzalkonium chloride, benzethonium, chlorohexidine, phenol, m-cresol, benzyl alcohol, methylparaben, propylparaben, chlorobutanol, o-cresol, p-cresol, chlorocresol, phenylmercuric nitrate, thimerosal, benzoic acid, and various mixtures thereof. In other embodiments, the pharmaceutical composition includes a bulking agent, like glycine. In yet other embodiments, the pharmaceutical composition includes a surfactant e.g., polysorbate-20, polysorbate-40, polysorbate- 60, polysorbate-65, polysorbate-80 polysorbate-85, poloxamer-188, sorbitan monolaurate, sorbitan monopalmitate, sorbitan monostearate, sorbitan monooleate, sorbitan trilaurate, sorbitan tristearate, sorbitan trioleaste, or a combination thereof. The pharmaceutical composition may also include a tonicity adjusting agent, e.g., a compound that renders the formulation substantially isotonic or isoosmotic with human blood. Exemplary tonicity adjusting agents include sucrose, sorbitol, glycine, methionine, mannitol, dextrose, inositol, sodium chloride, arginine and arginine hydrochloride. In other embodiments, the pharmaceutical composition additionally includes a stabilizer, e.g., a molecule which, when combined with a protein of interest substantially prevents or reduces chemical and/or physical instability of the protein of interest in lyophilized or liquid form. Exemplary stabilizers include sucrose, sorbitol, glycine, inositol, sodium chloride, methionine, arginine, and arginine hydrochloride.

In another embodiment, the GPBP-1 inhibitor is a compound of formula (A) as described in WO2011/054530: 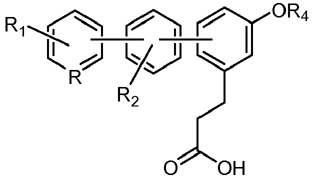 or a pharmaceutically acceptable salt thereof, wherein:
R is selected from N and CR₃; R₁ is hydrogen or C₁-C₆ alkoxy; R₂ is C₁-C₆ alkyl or halo(C₁-C₆ alkyl); R₃, if present, is C₁-C₆ alkyl or halo(C₁-C₆ alkyl); and R₄ is H or C₁-C₆ alkyl.

In another preferred embodiment of the compound of formula (A), R₁ is hydrogen or methoxy; R₂ is methyl, fluoromethyl, or difluoromethyl; R₃, if present, is methyl or trifluoromethyl; and R₄ is H or methyl.

In various further preferred embodiments, R₁ is hydrogen and/or R₄ is methyl. In further preferred embodiments, the compound of formula (A) is selected from the group consisting of: 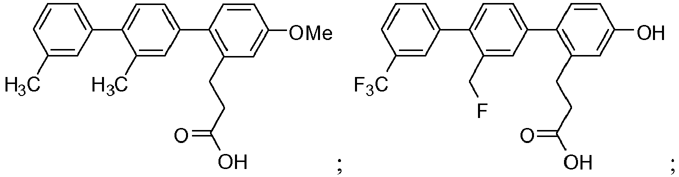 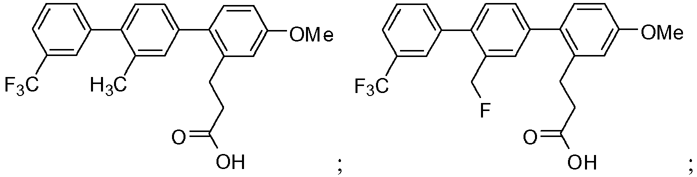 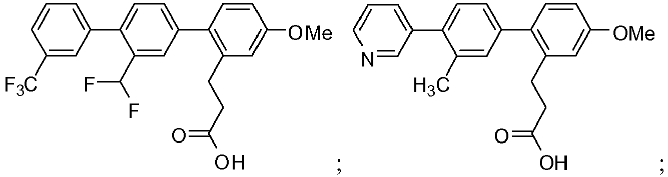 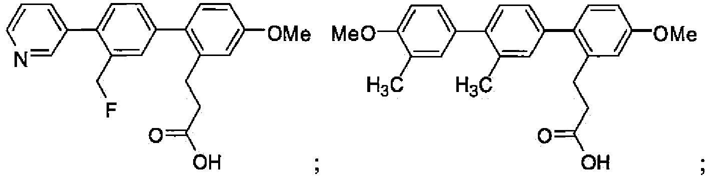 or a pharmaceutically acceptable salt thereof. In a most preferred embodiment, the compound is 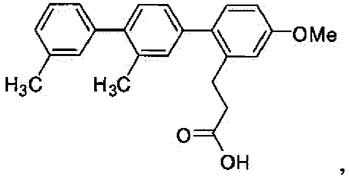 or a pharmaceutically acceptable salt thereof.

In another preferred embodiment, the GPBP-1 inhibitor is a compound of formula (B): 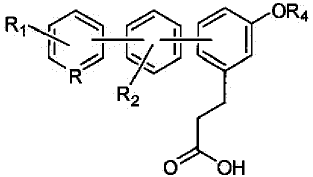 or a pharmaceutically acceptable salt thereof, wherein:
- R: is selected from N and CR₃;
R₃ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), -(CH₂)₁₋₅-C(O)NH₂, (aryl)C₂-C₆ alkyl, and (heteroaryl)C₁-C₆ alkyl;
- R₁: is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
- R₂: is C₁-C₆ alkyl, halo(C₁-C₆ alkyl), or hydroxy(C₁-C₆ alkyl); and
- R₄: is H, C₁-C₆ alkyl, -C(O)(C₁-C₂₀ alkyl), or -(CH₂)₁₋₅-C(O)OH.

In another embodiment the GPBP-1 inhibitor of formula B is a compound of formula (C): 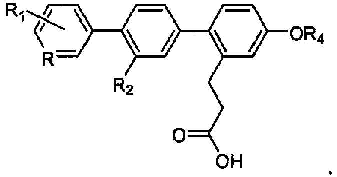 or a pharmaceutically acceptable salt thereof.

In one embodiment of the GPBP-1 inhibitors of formulae (B) or (C), R is selected from N and CR₃; R₃ is selected from the group consisting of hydrogen, halogen, hydroxy, C₁-C₆ alkyl, C₂-C₆ alkenyl, C₂-C₆ alkynyl, halo(C₁-C₆ alkyl), C₁-C₆ alkoxy, or halo(C₁-C₆ alkoxy; R₁ is hydrogen, hydroxy, C₁-C₆ alkyl, or C₁-C₆ alkoxy; R₂ is C₁-C₆ alkyl, halo(C₁-C₆ alkyl), or hydroxy(C₁-C₆ alkyl); and R₄ is H, C₁-C₆ alkyl, -C(O)(C₁-C₂₀ alkyl), or -(CH₂)₁₋₅-C(O)OH.

In another embodiment the GPBP-1 inhibitor is a compound having the formula:

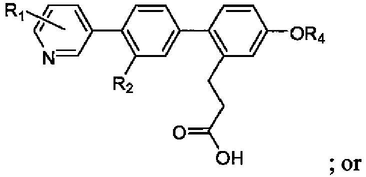

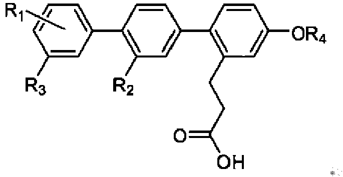

In various embodiments of any embodiment of the compounds of formulae (B) and (C), R₂ may be C₁-C₆ alkyl, halo(C₁-C₆ alkyl), or hydroxy(C₁-C₆ alkyl). For example, R₂ may be C₁-C₆ alkyl, methyl, halo(C₁-C₆ alkyl), fluoromethyl, difluoromethyl, hydroxymethyl, or hydroxy(C₁-C₆ alkyl).

In various further embodiments of any embodiment of the compounds of formulae (B) and (C), R₄ may be H, C₁-C₆ alkyl, methyl, propyl, -C(O)(C₁-C₂₀ alkyl), or -(CH₂)₁₋₅-C(O)OH.

In various further embodiments of any embodiment of the compounds of formulae (B) and (C), R₁ may be hydrogen or methoxy.

In various further embodiments of any embodiment of the compounds of formulae (B) and (C), R₃ may be C₁-C₆ alkyl, halo(C₁-C₆ alkyl), C₁-C₆ alkoxy, halo(C₁-C₆ alkoxy), methyl, or trifluoromethyl.

In a further embodiment of the compounds of formulae (B) and (C), R₁ is hydrogen or C₁-C₆ alkoxy; R₂ is hydroxy(C₁-C₆ alkyl); R₃, if present, is C₁-C₆ alkyl or halo(C₁-C₆ alkyl); and R₄ is H or C₁-C₆ alkyl.

In another embodiment of the compounds of formulae (B) and (C), R₁ is hydrogen or methoxy; R₂ is hydroxymethyl; R₃, if present, is methyl or trifluoromethyl; and R₄ is H or methyl.

In another embodiment the GPBP-1 inhibitor is a compound of formula (D): 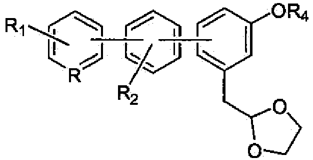 or a pharmaceutically acceptable salt thereof, wherein:
- R: is selected from N and CR₃;
R₃ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), -(CH₂)₁₋₅-C(O)NH₂, (aryl)C₂-C₆ alkyl, and (heteroaryl)C₁-C₆ alkyl;
- R₁: is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
- R₂: is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₁-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, or -CH=CH-C(O)(C₁-C₆ alkoxy); and
- R₄: is hydrogen, C₁-C₆ alkyl, or (aryl)C₁-C₆ alkyl.

In one embodiment, the compound of formula (D) is of the formula:

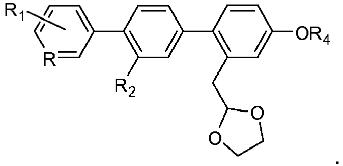

In one embodiment of any embodiment of the compound of formula (D), R₁ is hydrogen. In another embodiment of any embodiment of the compound of formula (D) R is CR₃, and R₃ is formyl(C₁-C₆ alkyl). In another embodiment, R₃ is -COH. In various embodiments, R₂ may be -CH=CH-C(O)OH; -CH=CH-C(O)(C₁-C₆ alkoxy); or -CH=CH-C(O)(OEt). In various further embodiments, R₄ may be (aryl)C₁-C₆ alkyl; or -CH₂-Ph.In a preferred embodiment, the compound of formula D is a compound is of the formula: 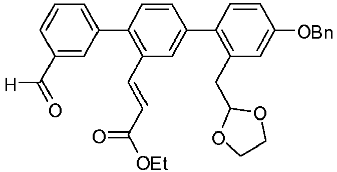 , or a pharmaceutically acceptable salt thereof.

In another embodiment, the GPBP-1 inhibitors comprise compounds of formula (I): 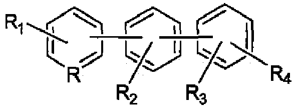 or a pharmaceutically acceptable salt thereof, wherein:
- R: is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), -(CH₂)₁₋₅-C(O)NH₂, (aryl)C₂-C₆ alkyl, and (heteroaryl)C₁-C₆ alkyl;
- R₁: is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
- R₂: is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, (aryl)C₁-C₆ alkyl, or (heteroaryl)C₁-C₆ alkyl;
- R₃: is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₁-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)-NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), (aryl)C₁-C₆ alkyl, or (heteroaryl)C₁-C₆ alkyl; and
- R₄: is hydroxy, halogen, C₁-C₆alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -O(CH₂)₁₋₅-C(O)OH, -O(CH₂)₁₋₅-C(O)(C₁-C₆ alkoxy), (aryl)C₁-C₆ alkyl, or (heteroaryl)C₁-C₆ alkyl.

In another embodiment, the GPBP-1 inhibitors are compounds of formula (II):

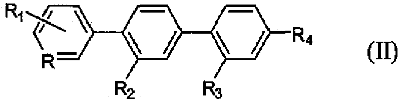

In another embodiment, the GPBP-1 inhibitors are compounds of formulae (I) or (II) wherein:
R is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), and -(CH₂)₁₋₅-C(O)NH₂;
R₁ is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
R₂ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), or -(CH₂)₁₋₅-C(O)NH₂;
R₃ is C₁-C₆ alkyl, hato(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₁-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, or -CH=CH-C(O)(C₁-C₆ alkoxy); and
R₄ is hydroxy, halogen, C₁-C₆alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -O(CH₂)₁₋₅-C(O)OH, or -O(CH₂)₁₋₅-C(O)(C₁-C₆ alkoxy).

In another embodiment, the GPBP-1 inhibitors are compounds of formulae (I) or (II) wherein:
R is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen, halogen, hydroxy, C₁-C₆ alkyl, halo(C₁-C₆ alkyl), C₁-C₆ alkoxy, or halo(C₁-C₆ alkoxy);
R₂ is C₁-C₆ alkyl, halo(C₁-C₆ alkyl), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), or (C₁-C₆ alkyl)sulfanyl(C₁-C₆ alkyl);
R3 is C₁-C₆ alkyl, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, or -CH=CH-C(O)(C₁-C₆ alkoxy); and
R₄ is hydroxy, halogen, C₁-C₆alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -O(CH₂)₁₋₅-C(O)OH, or -O(CH₂)₁₋₅-C(O)(C₁-C₆ alkoxy).

In another embodiment, the GPBP-1 inhibitors are compounds of formula (II), wherein R is N. These compounds can be represented by formula (III):

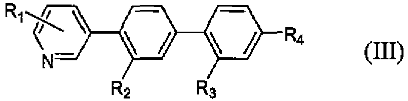

In yet another embodiment, the GPBP-1 inhibitors are compounds of formula (II), wherein R is CR₅. These compounds can be represented by formula (IV):

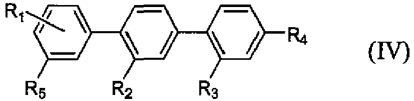

In one embodiment, the GPBP-1 inhibitors are compounds as described above with reference to any of formulae (I)-(IV), wherein R₁ is hydrogen, hydroxy, or C₁-C₆ alkoxy. In one embodiment, the GPBP-1 inhibitors are compounds as described above with reference to any of formulae (I)-(IV), wherein R₁ is hydrogen. In another embodiment, the GPBP-1 inhibitors are compounds as described above with reference to any of formulae (I)-(IV), wherein R₂ is C₁-C₆ alkyl, halo(C₁-C₆ alkyl), hydroxy(C₁-C₆ alkyl), formyl(C₀-C₆ alkyl), amino(C₁-C₆ alkyl), or sulfanyl(C₁-C₆ alkyl). In yet another embodiment, the GPBP-1 inhibitors are compounds as described above with reference to any of formulae (I)-(IV), wherein R₂ is C₁-C₆ alkyl, halo(C₁-C₆ alkyl), hydroxy(C₁-C₆ alkyl), or formyl(C₀-C₆ alkyl).

In yet another embodiment, the GPBP-1 inhibitors are compounds as described above with reference to any of formulae (I)-(IV), wherein R₂ can be C₁-C₆ alkyl, halo(C₁-C₆ alkyl), or hydroxy(C₁-C₆ alkyl). For example, in certain embodiments R₂ can be C₁-C₆ alkyl such as methyl, ethyl, or isopropyl. In other embodiments, R₂ can be halo(C₁-C₆ alkyl) such as fluoromethyl, difluoromethyl, or trifluoromethyl. R₂ can, in certain embodiments, be hydroxy(C₁-C₆ alkyl). For example, the hydroxy(C₁-C₆ alkyl) can be hydroxymethyl, 1-hydroxyethyl, or 2-hydroxyethyl.

In another embodiment, the GPBP-1 inhibitors are compounds as described above with reference to any of formulae (I)-(IV), wherein R₂ is C₁-C₆ alkyl. In certain embodiments R₂ is methyl. In another embodiment, the GPBP-1 inhibitors are compounds as described above with reference to any of formulae (I)-(IV), wherein R₃ is C₁-C₆ alkyl, -(CH₂)₁₋₅-C(O)OH, -(CH₂)₁₋₅-C(O)(C₁-C₆ alkoxy), -CH=CH-C(O)OH, or -CH=CH-C(O)(C₁-C₆ alkoxy). In another embodiment, the GPBP-1 inhibitors are compounds as described above with reference to any of formulae (I)-(IV), wherein R₃ is -(CH₂)₁₋₅-C(O)OH, -(CH₂)₁₋₅-C(O)(C₁-C₆ alkoxy), or -(CH₂)₁₋₅-C(O)NH₂.

In yet another embodiment, the GPBP-1 inhibitors are compounds as described above with reference to any of formulae (I)-(IV), wherein R₃ is -(CH₂)₁₋₂-C(O)OH, or -(CH₂)₁₋₂-C(O)(C₁-C₆ alkoxy). For example, in certain embodiments R₃ can be -(CH₂)₂-C(O)OH, -(CH₂)₂-C(O)(OCH₃), -(CH₂)₂-C(O)(OCH₂CH₃), or -(CH₂)₂-C(O)(OC(CH₃)₃). In other embodiments, R₃ can be -(CH₂)₂-C(O)OH, or -(CH₂)₂-C(O)(OCH₂CH₃).

In another embodiment, the GPBP-1 inhibitors are compounds as described above with reference to any of formulae (I)-(IV), wherein R₃ is -(CH₂)₁₋₂-C(O)OH. In one embodiment, R₃ is -(CH₂)₂-C(O)OH. In one embodiment, the GPBP-1 inhibitors are compounds as described above with reference to any of formulae (I)-(IV), wherein R₄ is hydroxy, halogen, C₁-C₆ alkyl, C₁-C₆ alkoxy, halo(C₁-C₆ alkoxy), or benzyloxy. In another embodiment, the GPBP-1 inhibitors are compounds as described above with reference to any of formulae (I)-(IV), wherein R₄ is hydroxy or C₁-C₆ alkoxy (e.g., methoxy). Preferably R₄ is C₁-C₆ alkoxy. In more preferred embodiment, R₄ is methoxy.

In one embodiment, the GPBP-1 inhibitors are compounds as described above with reference to formulae (I), (II), or (IV), wherein R₅ is C₁-C₆ alkyl, halo(C₁-C₆ alkyl), C₁-C₆ alkoxy, or halo(C₁-C₆ alkoxy). In another embodiment, the GPBP-1 inhibitors are compounds as described above with reference to formulae (I), (II), or (IV), wherein R₅ is C₁-C₆ alkyl, such as methyl. In yet another embodiment, the GPBP-1 inhibitors are compounds as described above with reference to formulae (I), (II), or (IV), wherein R₅ is halo(C₁-C₆ alkyl), such as trifluoromethyl.

In certain embodiments, the GPBP-1 inhibitors are compounds of any of formulae (I), (II), or (IV), wherein:
Rs is selected from the group consisting of hydrogen, halogen, cyano, nitro, hydroxy, C₁-C₆ alkyl, C₂-C₆ alkenyl, C2-C6 alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen, halogen, hydroxy, C₁-C₆ alkyl, halo(C₁-C₆ alkyl), C₁-C₆ alkoxy, or halo(C₁-C₆ alkoxy);
R₂ is C₁-C₆ alkyl, halo(C₁-C₆ alkyl), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), or (C₁-C₆ alkyl)thio(C₁-C₆ alkyl);
R3 is -(CH₂)₁₋₂-C(O)OH, -(CH₂)₁₋₂-C(O)(C₁-C₆ alkoxy), -(CH₂)₁₋₂-C(O)NH₂, -(CH₂)₁₋₂-C(O)NH(C₁-C₆ alkyl), -(CH₂)₁₋₂-C(O)N(C₁-C₆ alkyl)₂, -CH=CH-C(O)OH, -CH=CH-C(O)(C₁-C₆ alkoxy); and
R₄ is hydroxy, C₁-C₆ alkoxy, halo(C₁-C₆ alkoxy), or benzyloxy.

In certain embodiments, the GPBP-1 inhibitors are compounds of any of formula (III), wherein:
R₁ is hydrogen, halogen, hydroxy, C₁-C₆ alkyl, halo(C₁-C₆ alkyl), C₁-C₆ alkoxy, or halo(C₁-C₆ alkoxy);
R₂ is C₁-C₆ alkyl, halo(C₁-C₆ alkyl), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), or (C₁-C₆ alkyl)thio(C₁-C₆ alkyl);
R3 is -(CH₂)₁₋₂-C(O)OH, -(CH₂)₁₋₂-C(O)(C₁-C₆ alkoxy), -(CH₂)₁₋₂-C(O)NH₂, -(CH₂)₁₋₂-C(O)NH(C₁-C₆ alkyl), -(CH₂)₁₋₂-C(O)N(C₁-C₆ alkyl)₂, -CH=CH-C(O)OH, -CH=CH-C(O)(C₁-C₆ alkoxy); and
R₄ is hydroxy, C₁-C₆ alkoxy, halo(C₁-C₆ alkoxy), or benzyloxy.

In certain embodiments, the GPBP-1 inhibitors are compounds of any of formulae (I), (II), or (IV), wherein R₁ is hydrogen; R₂ is C₁-C₆ alkyl, halo(C₁-C₆ alkyl), hydroxy(C₁-C₆ alkyl), or formyl(C₁-C₆ alkyl); R₃ is -(CH₂)₁₋₂-C(O)OH, -(CH₂)₁₋₂-C(O)(C₁-C₆ alkoxy), or -(CH₂)₁₋₂-C(O)NH₂; R₄ is hydroxy or C₁-C₆ alkoxy; and R₅ is C₁-C₆ alkyl, halo(C₁-C₆ alkyl), C₁-C₆ alkoxy, or halo(C₁-C₆ alkoxy).

In certain embodiments, the GPBP-1 inhibitors are compounds of any of formula (III), wherein R₁ is hydrogen; R₂ is C₁-C₆ alkyl, halo(C₁-C₆ alkyl), hydroxy(C₁-C₆ alkyl), or formyl(C₁-C₆ alkyl); R₃ is -(CH₂)₁₋₂-C(O)OH, -(CH₂)₁₋₂-C(O)(C₁-C₆ alkoxy), or -(CH₂)₁₋₂-C(O)NH₂; R₄ is hydroxy or C₁-C₆ alkoxy; and R₅ is C₁-C₆ alkyl, halo(C₁-C₆ alkyl), C₁-C₆ alkoxy, or halo(C₁-C₆ alkoxy).

In certain embodiments, the GPBP-1 inhibitors are compounds of any of formulae (I)-(IV), wherein:
R, if present, is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen;
R₂ is C₁-C₆ alkyl;
R₃ is -(CH₂)₁₋₂-C(O)OH; and
R₄ is C₁-C₆ alkoxy.

In certain embodiments, the GPBP-1 inhibitors are compounds of any of formulae (I)-(IV), wherein:
R, if present, is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen;
R₂ is methyl;
R₃ is -(CH₂)₂-C(O)OH; and
R₄ is methoxy.

In one embodiment, the GPBP-1 inhibitors are compounds of formula (V): 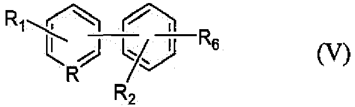 or a pharmaceutically acceptable salt thereof, wherein:
R is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, (aryl)C₁-C₆ alkyl, and (heteroaryl)C₁-C₆ alkyl;
R₁ is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
R₂ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), (aryl)C₁-C₆ alkyl, or (heteroaryl)C₁-C₆ alkyl; and
R₆ is hydroxy, halogen, C₁-C₆ alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -OS(O)₂CF₃, (aryl)C₁-C₆ alkyl, or (heteroaryl)C₁-C₆ alkyl.

In one embodiment, the compounds of formula (V) are of formula (VI):

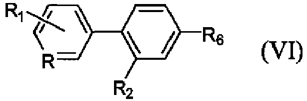

In another embodiment, the GPBP-1 inhibitors are compounds of formulae (V) or (VI) wherein:
R is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), and -(CH₂)₁₋₅-C(O)NH₂;
R₁ is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
R₂ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -CH=CH-C(O)OH, or -CH=CH-C(O)(C₁-C₆ alkoxy); and R₆ is hydroxy, halogen, C₁-C₆ alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₆-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), or -OS(O)₂CF₃.

In another embodiment, the GPBP-1 inhibitors are compounds of formulae (V) or (VI) wherein:
R is selected from N and CR₅;
Rs is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen, halogen, hydroxy, C₁-C₆ alkyl, halo(C₁-C₆ alkyl), C₁-C₆ alkoxy, or halo(C₁-C₆ alkoxy);
R₂ is C₁-C₆ alkyl, halo(C₁-C₆ alkyl), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -CH=CH-C(O)OH, or -CH=CH-C(O)(C₁-C₆ alkoxy); and
R₆ is hydroxy, halogen, C₁-C₆ alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), or -OS(O)₂CF₃.

In one embodiment, the GPBP-1 inhibitors are compounds as described above with reference to any of formulae (V)-(VI), wherein R₁ is hydrogen.

In another embodiment, the GPBP-1 inhibitors are compounds as described above with reference to any of formulae (V)-(VI), wherein R₂ is C₁-C₆ alkyl, halo(C₁-C₆ alkyl), hydroxy(C₁-C₆ alkyl), formyl(C₀-C₆ alkyl), amino(C₁-C₆ alkyl), sulfanyl(C₁-C₆ alkyl), -CH=CH-C(O)OH, or -CH=CH-C(O)(C₁-C₆ alkoxy).

In yet another embodiment, the GPBP-1 inhibitors are compounds as described above with reference to any of formulae (V)-(VI), wherein R₂ is C₁-C₆ alkyl, halo(C₁-C₆ alkyl), hydroxy(C₁-C₆ alkyl), formyl(C₀-C₆ alkyl), -CH=CH-C(O)OH, or -CH=CH-C(O)(C₁-C₆ alkoxy).

In yet another embodiment, the GPBP-1 inhibitors are compounds as described above with reference to any of formulae (V)-(VI), wherein R₂ can be C₁-C₆ alkyl, halo(C₁-C₆ alkyl), hydroxy(C₁-C₆ alkyl), or -CH=CH-C(O)(C₁-C₆ alkoxy). For example, in certain embodiments R₂ can be C₁-C₆ alkyl such as methyl, ethyl, or isopropyl. In other embodiments, R₂ can be halo(C₁-C₆ alkyl) such as fluoromethyl, difluoromethyl, or trifluoromethyl. R₂ can, in certain embodiments, be hydroxy(C₁-C₆ alkyl). For example, the hydroxy(C₁-C₆ alkyl) can be hydroxymethyl, 1-hydroxyethyl, or 2-hydroxyethyl.

In another embodiment, the GPBP-1 inhibitors are compounds as described above with reference to any of formulae (V)-(VI), wherein R₂ is C₁-C₆ alkyl. In certain embodiments R₂ is methyl.

In one embodiment, the GPBP-1 inhibitors are compounds as described above with reference to any of formulae (V)-(VI), wherein R₆ is hydroxy, halogen, C₁-C₆ alkyl, C₁-C₆ alkoxy, halo(C₁-C₆ alkoxy), benzyloxy, or -OS(O)₂CF₃.

In another embodiment, the GPBP-1 inhibitors are compounds as described above with reference to any of formulae (V)-(VI), wherein R₆ is hydroxy or C₁-C₆ alkoxy (e.g., methoxy). In one embodiment, the GPBP-1 inhibitors are compounds as described above with reference to formulae (V)-(VI), wherein R₅ is C₁-C₆ alkyl, halo(C₁-C₆ alkyl), C₁-C₆ alkoxy, or halo(C₁-C₆ alkoxy). In another embodiment, the GPBP-1 inhibitors are compounds as described above with reference to formulae (V)-(VI), wherein R₅ is C₁-C₆ alkyl, such as methyl. In yet another embodiment, the GPBP-1 inhibitors are compounds as described above with reference to formulae (V)-(VI), wherein R₅ is halo(C₁-C₆ alkyl), such as trifluoromethyl.

In certain embodiments, the GPBP-1 inhibitors are compounds of any of formulae (V)-(VI), wherein:
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, and amino(C₁-C₆ alkyl);
R₁ is hydrogen, halogen, hydroxy, C₁-C₆ alkyl, halo(C₁-C₆ alkyl), C₁-C₆ alkoxy, or halo(C₁-C₆ alkoxy);
R₂ is C₁-C₆ alkyl, halo(C₁-C₆ alkyl), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)thio(C₁-C₆ alkyl), or -CH=CH-C(O)(C₁-C₆ alkoxy); and
R₆ is hydroxy, C₁-C₆ alkoxy, halo(C₁-C₆ alkoxy), or -OS(O)₂CF₃.

In certain embodiments, the GPBP-1 inhibitors are compounds of any of formulae (V)-(VI), wherein R₁ is hydrogen; R₂ is C₁-C₆ alkyl, halo(C₁-C₆ alkyl), hydroxy(C₁-C₆ alkyl), formyl(C₀-C₆ alkyl), or -CH=CH-C(O)(C₁-C₆ alkoxy); R₆ is hydroxy, C₁-C₆ alkoxy, or -OS(O)₂CF₃; and R₅ is C₁-C₆ alkyl, halo(C₁-C₆ alkyl), C₁-C₆ alkoxy, or halo(C₁-C₆ alkoxy).

Synthesis of the other GPBP-1 inhibitors disclosed herein can be carried out by those of skill in the art, based on the teachings in WO2011/054530.

In one preferred embodiment, the GPBP-1 inhibitor comprises or consists of 3-[4"-methoxy-3,2'-dimethyl-(1,1';4',1")terphenyl-2"-yl]propionic acid; 3-[2'-methyl-4"-methoxy-3-(trifluoromethyl)-(1,1';4',1")terphenyl-2"-yl]propionic acid; combinations thereof, or a pharmaceutically acceptable salt thereof.

The GBPB-1 inhibitor compounds include pharmaceutically acceptable salts, esters, amides, and prodrugs thereof, including but not limited to carboxylate salts, amino acid addition salts, esters, amides, and prodrugs of the compounds of the present invention which are, within the scope of sound medical judgment, suitable for use in contact with the tissues of patients without undue toxicity, irritation, allergic response, and the like, commensurate with a reasonable benefit/risk ratio, and effective for their intended use, as well as the zwitterionic forms, where possible, of the compounds of the invention. The term "salts" refers to the relatively non-toxic, inorganic and organic acid addition salts of compounds of the present invention. These salts can be prepared *in situ* during the final isolation and purification of the compounds or by separately reacting the purified compound in its free base form with a suitable organic or inorganic acid and isolating the salt thus formed. Representative salts include the hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. These may include cations based on the alkali and alkaline earth metals, such as sodium, lithium, potassium, calcium, magnesium, and the like, as well as non-toxic ammonium, quaternary ammonium, and amine cations including, but not limited to ammonium, tetramethylammonium, tetraethylammonium, methylamine, dimethylamine, trimethylamine, triethylamine, ethylamine, and the like. (See, for example, Berge S.M. et al, "Pharmaceutical Salts," 1977.J. Pharm. Sci.,66: 1-19.)

Examples of pharmaceutically acceptable, non-toxic esters of the inhibitors include C₁-C₆ alkyl esters, wherein the alkyl group is straight or branched, substituted or unsubstituted, C₅-C₇ cycloalkyl esters, as well as arylalkyl esters such as benzyl and triphenylmethyl. C₁-C₄ alkyl esters are preferred, such as methyl, ethyl, 2,2,2-trichloroethyl, and tert-butyl. Esters of the compounds of the present invention may be prepared according to conventional methods.

Examples of pharmaceutically acceptable, non-toxic amides of the inhibitors include amides derived from ammonia, primary C₁-C₆ alkyl amines and secondary C₁-C₆ dialkyl amines, wherein the alkyl groups are straight or branched. In the case of secondary amines, the amine may also be in the form of a 5- or 6-membered heterocycle containing one nitrogen atom. Amides derived from ammonia, C₁-C₃ alkyl primary amines and C₁-C₂ dialkyl secondary amines are preferred. Amides of the compounds of the invention may be prepared according to conventional methods.

The term "prodrug" refers to compounds that are rapidly transformed *in vivo* to yield the parent compound of the above formulae, for example, by hydrolysis in blood. A thorough discussion of prodrugs is provided in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

The term "alkenyl" as used herein, means a straight or branched chain hydrocarbon containing from 2 to 10 carbons, unless otherwise specified, and containing at least one carbon-carbon double bond. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, 3-decenyl, and 3,7-dimethylocta-2,6-dienyl.

The term "alkoxy" as used herein, means an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkyl" as used herein, means a straight or branched chain hydrocarbon containing from 1 to 10 carbon atoms unless otherwise specified. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl. When an "alkyl" group is a linking group between two other moieties, then it may also be a straight or branched chain; examples include, but are not limited to -CH₂-, -CH₂CH₂-, -CH₂CH₂CHC(CH₃)-, -CH₂CH(CH₂CH₃)CH₂-.

The term "alkylene" refers to a bivalent alkyl group. An "alkylene chain" is a polymethylene group, i.e., -(CH₂)ₙ-, wherein n is a positive integer, preferably from one to six, from one to four, from one to three, from one to two, or from two to three. A substituted alkylene chain is a polymethylene group in which one or more methylene hydrogen atoms is replaced with a substituent. Suitable substituents include those described below for a substituted aliphatic group. An alkylene chain also may be substituted at one or more positions with an aliphatic group or a substituted aliphatic group.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "aryl," as used herein, means a phenyl (*i.e.,* monocyclic aryl), or a bicyclic ring system containing at least one phenyl ring or an aromatic bicyclic ring containing only carbon atoms in the aromatic bicyclic ring system. The bicyclic aryl can be azulenyl, naphthyl, or a phenyl fused to a monocyclic cycloalkyl, a monocyclic cycloalkenyl, or a monocyclic heterocyclyl. The bicyclic aryl is attached to the parent molecular moiety through any carbon atom contained within the phenyl portion of the bicyclic system, or any carbon atom with the napthyl or azulenyl ring. The fused monocyclic cycloalkyl or monocyclic heterocyclyl portions of the bicyclic aryl are optionally substituted with one or two oxo and/or thia groups. Representative examples of the bicyclic aryls include, but are not limited to, azulenyl, naphthyl, dihydroinden-1-yl, dihydroinden-2-yl, dihydroinden-3-yl, dihydroinden-4-yl, 2,3-dihydroindol-4-yl, 2,3-dihydroindol-5-yl, 2,3-dihydroindol-6-yl, 2,3-dihydroindol-7-yl, inden-1-yl, inden-2-yl, inden-3-yl, inden-4-yl, dihydronaphthalen-2-yl, dihydronaphthalen-3-yl, dihydronaphthalen-4-yl, dihydronaphthalen-1-yl, 5,6,7,8-tetrahydronaphthalen-1-yl, 5,6,7,8-tetrahydronaphthalen-2-yl, 2,3-dihydrobenzofuran-4-yl, 2,3-dihydrobenzofuran-5-yl, 2,3-dihydrobenzofuran-6-yl, 2,3-dihydrobenzofuran-7-yl, benzo[d][1,3]dioxol-4-yl, benzo[d][1,3]dioxol-5-yl, 2H-chromen-2-on-5-yl, 2H-chromen-2-on-6-yl, 2H-chromen-2-on-7-yl, 2H-chromen-2-on-8-yl, isoindoline-1,3-dion-4-yl, isoindoline-1,3-dion-5-yl, inden-1-on-4-yl, inden-1-on-5-yl, inden-1-on-6-yl, inden-1-on-7-yl, 2,3-dihydrobenzo[b][1,4]dioxan-5-yl, 2,3-dihydrobenzo[b][1,4]dioxan-6-yl, 2H-benzo[b][1,4]oxazin3(4H)-on-5-yl, 2H-benzo[b][1,4]oxazin3(4H)-on-6-yl, 2H-benzo[b][1,4]oxazin3(4H)-on-7-yl, 2H-benzo[b][1,4]oxazin3(4H)-on-8-yl, benzo[d]oxazin-2(3H)-on-5-yl, benzo[d]oxazin-2(3H)-on-6-yl, benzo[d]oxazin-2(3H)-on-7-yl, benzo[d]oxazin-2(3H)-on-8-yl, quinazolin-4(3H)-on-5-yl, quinazolin-4(3H)-on-6-yl, quinazolin-4(3H)-on-7-yl, quinazolin-4(3H)-on-8-yl, quinoxalin-2(1H)-on-5-yl, quinoxalin-2(1H)-on-6-yl, quinoxalin-2(1H)-on-7-yl, quinoxalin-2(1H)-on-8-yl, benzo[d]thiazol-2(3H)-on-4-yl, benzo[d]thiazol-2(3H)-on-5-yl, benzo[d]thiazol-2(3H)-on-6-yl, and, benzo[d]thiazol-2(3H)-on-7-yl. In certain embodiments, the bicyclic aryl is (i) naphthyl or (ii) a phenyl ring fused to either a 5 or 6 membered monocyclic cycloalkyl, a 5 or 6 membered monocyclic cycloalkenyl, or a 5 or 6 membered monocyclic heterocyclyl, wherein the fused cycloalkyl, cycloalkenyl, and heterocyclyl groups are optionally substituted with one or two groups which are independently oxo or thia.

The term "halo" or "halogen" as used herein, means -Cl, -Br, -I or -F.

The terms "haloalkyl", "haloalkenyl" and "haloalkoxy" refer to an alkyl, alkenyl or alkoxy group, as the case may be, which is substituted with one or more halogen atoms.

The term "heteroaryl," as used herein, means a monocyclic heteroaryl or a bicyclic ring system containing at least one heteroaromatic ring. The monocyclic heteroaryl can be a 5 or 6 membered ring. The 5 membered ring consists of two double bonds and one, two, three or four nitrogen atoms and optionally one oxygen or sulfur atom. The 6 membered ring consists of three double bonds and one, two, three or four nitrogen atoms. The 5 or 6 membered heteroaryl is connected to the parent molecular moiety through any carbon atom or any nitrogen atom contained within the heteroaryl. Representative examples of monocyclic heteroaryl include, but are not limited to, furyl, imidazolyl, isoxazolyl, isothiazolyl, oxadiazolyl, oxazolyl, pyridinyl, pyridazinyl, pyrimidinyl, pyrazinyl, pyrazolyl, pyrrolyl, tetrazolyl, thiadiazolyl, thiazolyl, thienyl, triazolyl, and triazinyl. The bicyclic heteroaryl consists of a monocyclic heteroaryl fused to a phenyl, a monocyclic cycloalkyl, a monocyclic cycloalkenyl, a monocyclic heterocyclyl, or a monocyclic heteroaryl. The fused cycloalkyl or heterocyclyl portion of the bicyclic heteroaryl group is optionally substituted with one or two groups which are independently oxo or thia. When the bicyclic heteroaryl contains a fused cycloalkyl, cycloalkenyl, or heterocyclyl ring, then the bicyclic heteroaryl group is connected to the parent molecular moiety through any carbon or nitrogen atom contained within the monocyclic heteroaryl portion of the bicyclic ring system. When the bicyclic heteroaryl is a monocyclic heteroaryl fused to a benzo ring, then the bicyclic heteroaryl group is connected to the parent molecular moiety through any carbon atom or nitrogen atom within the bicyclic ring system. Representative examples of bicyclic heteroaryl include, but are not limited to, benzimidazolyl, benzofuranyl, benzothienyl, benzoxadiazolyl, benzoxathiadiazolyl, benzothiazolyl, cinnolinyl, 5,6-dihydroquinolin-2-yl, 5,6-dihydroisoquinolin-1-yl, furopyridinyl, indazolyl, indolyl, isoquinolinyl, naphthyridinyl, quinolinyl, purinyl, 5,6,7,8-tetrahydroquinolin-2-yl, 5,6,7,8-tetrahydroquinolin-3-yl, 5,6,7,8-tetrahydroquinolin-4-yl, 5,6,7,8-tetrahydroisoquinolin-1-yl, thienopyridinyl, 4,5,6,7-tetrahydrobenzo[c][1,2,5]oxadiazolyl, and 6,7-dihydrobenzo[c][1,2,5]oxadiazol-4(5H)-onyl. In certain embodiments, the fused bicyclic heteroaryl is a 5 or 6 membered monocyclic heteroaryl ring fused to either a phenyl ring, a 5 or 6 membered monocyclic cycloalkyl, a 5 or 6 membered monocyclic cycloalkenyl, a 5 or 6 membered monocyclic heterocyclyl, or a 5 or 6 membered monocyclic heteroaryl, wherein the fused cycloalkyl, cycloalkenyl, and heterocyclyl groups are optionally substituted with one or two groups which are independently oxo or thia.

The GPBP-1 inhibitors are ordinarily combined with one or more adjuvants appropriate for the indicated route of administration. The inhibitors may be mixed with lactose, sucrose, starch powder, cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol, and tableted or encapsulated for conventional administration. Alternatively, the inhibitors may be dissolved in saline, water, polyethylene glycol, propylene glycol, carboxymethyl cellulose colloidal solutions, ethanol, corn oil, peanut oil, cottonseed oil, sesame oil, tragacanth gum, and/or various buffers. Other adjuvants and modes of administration are well known in the pharmaceutical art. The carrier or diluent may include time delay material, such as glyceryl monostearate or glyceryl distearate alone or with a wax, or other materials well known in the art.

The GPBP-1 inhibitors can be administered as the sole active pharmaceutical agent, or they can be used in combination with one or more other compounds useful for carrying out the methods of the invention. When administered as a combination, the therapeutic agents can be formulated as separate compositions that are given at the same time or different times, or the therapeutic agents can be given as a single composition.

The GPBP-1 inhibitors may be made up in a solid form (including granules, powders or suppositories) or in a liquid form (*e.g.,* solutions, suspensions, or emulsions).

The GPBP-1 inhibitors may be applied in a variety of solutions and may be subjected to conventional pharmaceutical operations such as sterilization and/or may contain conventional adjuvants, such as preservatives, stabilizers, wetting agents, emulsifiers, buffers etc.

The GPBP-1 inhibitors maybe administered orally, topically, parenterally, by inhalation or spray or rectally in dosage unit formulations containing conventional non-toxic pharmaceutically acceptable carriers, adjuvants and vehicles. The term parenteral as used herein includes percutaneous, subcutaneous, intravascular (e.g., intravenous), intramuscular, or intrathecal injection or infusion techniques and the like. The pharmaceutical compositions containing GPBP-1 inhibitors may be in a form suitable for oral use, for example, as tablets, troches, lozenges, aqueous or oily suspensions, dispersible powders or granules, emulsion, hard or soft capsules, or syrups or elixirs.

Compositions intended for oral use may be prepared according to any method known to the art for the manufacture of pharmaceutical compositions and such compositions may contain one or more agents selected from the group consisting of sweetening agents, flavoring agents, coloring agents and preservative agents in order to provide palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques. In some cases such coatings may be prepared by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydropropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Oily suspensions may be formulated by suspending the inhibitors in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents and flavoring agents may be added to provide palatable oral preparations. These compositions may be preserved by the addition of an anti-oxidant such as ascorbic acid.

Dispersible powders and granules suitable for preparation of an aqueous suspension by the addition of water provide the GPBP-1 inhibitors in admixture with a dispersing or wetting agent, suspending agent and one or more preservatives. Suitable dispersing or wetting agents or suspending agents are exemplified by those already mentioned above. Additional excipients, for example sweetening, flavoring and coloring agents, may also be present.

GPBP-1 inhibitors may also be administered in the form of oil-in-water emulsions. The oily phase may be a vegetable oil or a mineral oil or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol, glucose or sucrose. Such formulations may also contain a demulcent, a preservative, and flavoring and coloring agents. The GPBP-1 inhibitor compositions may be in the form of a sterile injectable aqueous or oleaginous suspension. This suspension may be formulated according to the known art using those suitable dispersing or wetting agents and suspending agents that have been mentioned above. The sterile injectable preparation may also be a sterile injectable solution or suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono-or diglycerides. In addition, fatty acids such as oleic acid find use in the preparation of injectables.

The GPBP-1 inhibitor-containing compositions may also be administered in the form of suppositories, e.g., for rectal administration of the drug. These compositions can be prepared by mixing the drug with a suitable non-irritating excipient that is solid at ordinary temperatures but liquid at the rectal temperature and will therefore melt in the rectum to release the drug. Such materials include cocoa butter and polyethylene glycols.

The GPBP-1 inhibitor-containing compositions of the present invention may be administered parenterally in a sterile medium. The drug, depending on the vehicle and concentration used, can either be suspended or dissolved in the vehicle. Advantageously, adjuvants such as local anesthetics, preservatives and buffering agents can be dissolved in the vehicle.

Dosage levels of GPBP-1 inhibitors on the order of from about 0.01 mg to about 50 mg per kilogram of body weight per day, and more preferably between 0.1 mg to about 50 mg per kilogram of body weight per day, are useful in the treatment of the above-indicated conditions. The amount of GPBP-1 inhibitor that may be combined with the carrier materials to produce a single dosage form will vary depending upon the host treated and the particular mode of administration. Dosage unit forms will generally contain between from about 1 mg to about 500 mg of inhibitor.

"Pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problems or complications commensurate with a reasonable benefit/risk ratio or which have otherwise been approved by the United States Food and Drug Administration as being acceptable for use in humans or domestic animals.

In one embodiment of any therapeutic aspect of the invention, the GPBP-1 inhibitor(s) may be co-administered with one or more other therapeutics for treating T2D and diabetic complications, including but not limited to insulin, metformin, sulfonylureas, alpha glucosidase inhibitors, thiazolidendiones, glucagon-like peptide-1 and analogs thereof, dipeptidyl peptidase-4 inhibitors, and angiotensin-converting enzyme inhibitors (ACEIs).

In another aspect, the present invention provides methods for diagnosing a pre-diabetic state comprising
(a) determining an amount of GPBP-1 in a sample from a subject at risk of having a pre-diabetic state; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample indicates the presence of a pre-diabetic state in the subject.

As shown in the examples that follow, circulating levels GPBP were significantly increased in a normoglycemic *Irs2*^{-/-}mice (model of T2D) as compared with wild-type (WT). However, in diabetic *Irs2*^{-/-}mice, a marked reduction in circulating levels of GPBP was observed, demonstrating that up-regulation of GPBP occurs in the pre-diabetic state and predicts T2D development. Thus, GPBP is a biomarker of the pre-diabetic state that can be used clinically for the early detection of insulin resistance and propensity to develop T2D.

As used herein, a "pre-diabetic state" is the state in which some but not all of the diagnostic criteria for diabetes are met. Thus, the pre-diabetic state may comprise: 1) having impaired fasting glucose tolerance, which is a condition whereby the response of beta cells to an oral glucose challenge (OGT) is deficient or 2) having consistently elevated fasting glucose (IFG), which is a condition in which the fasting blood glucose is elevated above what is considered normal levels but is not high enough to be classified as diabetes mellitus. The pre-diabetic state may be associated with insulin resistance and increased risk of cardiovascular pathology. Individuals with a pre-diabetic state are at a relatively high risk of developing T2D. In a further aspect, the invention provides methods for diagnosing a propensity to develop T2D, comprising
(a) determining an amount of GPBP-1 in a sample from a subject at risk of developing T2D; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample correlates with a propensity to develop T2D in the subject.

Such "increase" can be any amount of increase in GPBP-1 relative to control (such as control sample from normal subject, or previously determined "normal" levels of GPBP-1 in a control polupation), for example, 5%, 10%, 15%, 20%, 25%, 50%, 75%, 100%, or greater. In one embodiment, a normal value of GPBP-1 as a reference for a standard curve is less than 10 ng/ml in plasma. In various embodiments, a normal GPBP-1 range may be between ∼ 1 ng/ml-10 ng/ml in plasma. Thus, in one embodiment, subjects identified as having more than 10 ng/ml of GPBP-1 in their plasma are treated according to the methods of the invention. Methods for determining the amount of circulating GPBP are known (see WO 2010/009856 and US 7935492).

As used herein, a 'propensity to develop T2D means that the subject has an increased risk of developing T2D compared to the general population.

In these embodiments, any suitable sample from a subject may be used as disclosed herein, including but not limited to a serum sample.

Subjects at risk of a pre-diabetic state and/or at risk of developing T2D include, but are not limited to, those subjects with one or more risk factors selected including, but not limited to obesity, a sedentary lifestyle, poor eating habits (ex: too much fat, not enough fiber, too many simple carbohydrates, etc.), family history of pre-diabetic state and/or T2D, age 50 or older, high blood pressure, high cholesterol, and history of gestational diabetes.

In one embodiment, the diagnosing comprises
(a) contacting a plasma sample from the subject with a GPBP-1 binding molecule that binds to GPBP-1 under conditions to promote selective binding of the GPBP-1 binding molecule to the GPBP-1;
(b) removing unbound plasma and/or GPBP-1 binding molecules; and
(c) detecting complex formation between the GPBP-1 binding molecule and the GPBP-1 in the plasma sample, wherein complex formation provides a measure of GPBP-1 in the sample.

A "GPBP binding molecule" is a peptide or nucleic acid molecule that binds selectively to GPBP, as opposed to one or more other biological molecules, structures, cells, tissues, etc. Exemplary embodiments of such GPBP binding molecules include but are not limited to antibodies, aptamers or substrates. As used herein, a "GPBP substrate" is a target of GPBP biological activity that binds to GPBP, or a fragment thereof that retains GPBP binding activity. Such GPBP substrates include, but are not limited to, I-20 (SEQ ID NO:9), GPBP-interacting proteins (GIPs) (SEQ ID NOS:14-18), myelin basic protein (MBP) and derivatives thereof (SEQ ID NOS: 19-22), prion protein (PrP) (SEQ ID NO:23), α3NC1(SEQ ID NO:24), and Alzheimer's disease beta peptide (Aβ₁₋₄₂) (SEQ ID NO:25). Exemplary references demonstrating GPBP binding of these substrates can be found in US Patent Nos. 6,579,969; 7,147,855; and 7,326,768.

A "plasma sample" means blood plasma, the liquid component of blood, and is prepared, for example, by centrifugation of whole blood to remove blood cells. As used herein, a plasma sample also includes a blood serum sample, in which blood clotting factors have been removed.

The plasma sample may be obtained from any suitable subject, preferably from a mammal that is at risk of T2D or pre-diabetes, including but not limited to a human, dog, cat, horse, or livestock (cow, sheep, etc.). In a most preferred embodiment, the plasma sample is obtained from a human subject.

The antibody can be any selective GPBP-1 antibody, whether polyclonal, monoclonal, or humanized monoclonal as described above, although monoclonal antibodies are preferred.

Conditions suitable to promote binding of GPBP-binding molecules, such as antibodies, aptamers or substrates, to GPBP in the plasma samples can be determined by those of skill in the art based on the teachings herein and the examples provided below. For example, antibody-antigen binding often depends on hydrophobic interactions (the so called hydrophobic bonds); thus, high salt concentrations, such as in the molar range can be used to reduce nonspecific binding and increase specific antigen-antibody binding. Optionally, further steps may be included to promote selectivity and specificity, including but not limited to one or more wash steps to remove unbound GPBP and/or GPBP-binding molecule, or unbound or weakly bound serum proteins; inhibitors of non-specific binding to reduce binding of high concentration serum proteins, control samples known to contain GPBP and/or negative controls known not to bind to GPBP, and/or inclusion of plasma samples known to not possess GPBP (ex: deleted for GPBP).

The methods can test for the presence of GPBP in the plasma sample by standard techniques including, but not limited to ELISA, immunoflourescence, and chromatography (for example, lateral flow assays where the antibody is immobilized on a surface and plasma proteins are labeled and allowed to flow over the surface under conditions suitable to permit binding of the antibody to GPBP in the plasma). In one embodiment, functional beads (Becton Dickinson technology) coupled to flow cytometry are used; this technique is an emerging method to measure the levels of proteins in biological fluid or cell/tissue extracts. Specifically, beads made of a fluorescence matrix are coated with one or more GPBP-specific antibodies, mixed with the plasma sample and further incubated with a detecting GPBP antibody labeled with a phycoerythrins. Finally, beads are analyzed by a flow cytometry program which selects the beads according matrix fluorescence emission and measurement of the level of the analyte through phycoerythrin emission. There are up to thirty different types of beads that can be simultaneously detected and discriminated by the cytometer. In another embodiment, microspheres (x MAP technology) coupled to flow cytometry or to CCD imaging based technology (multiplexing platforms) are used. Specifically, microspheres made of polystyrene and internally dyed with red and infrared fluorophores are coated with one or more GPBP-specific antibodies, mixed with the plasma sample, further incubated with a detecting GPBP antibody labeled with biotin and finally complexes are detected with streptavidin labeled with phycoerythrin. Beads are subsequently analyzed by multiplexing platforms which selects the beads according to fluorescence emission and measurement of the level of the analyte through phycoerythrin emission. There are up to five hundred different types of microspheres that can be simultaneously detected and discriminated by the multiplexing platforms. These methods couple high sensitivity and performance with versatility since a specific functional bead or a microsphere coated with GPBP- specific antibodies can be mixed with a distinct bead type coated with binding peptides for other analyte (i.e. autoantibodies) and simultaneously measured. The measurement of various analytes could enhance the potential of GPBP determination. In one embodiment, the techniques may determine only the presence or absence of the GPBP. Alternatively, the techniques may be quantitative, and provide information about the relative amount of the GPBP in the sample. For quantitative purposes, ELISAs are preferred.

Detection of immunocomplex formation can be accomplished by standard detection techniques. For example, detection of immunocomplexes can be accomplished by using labeled antibodies or secondary antibodies. Such methods, including the choice of label are known to those ordinarily skilled in the art. Alternatively, the antibodies can be coupled to a detectable substance. The term "coupled" is used to mean that the detectable substance is physically linked to the antibody. Suitable detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase. Examples of suitable prosthetic-group complexes include streptavidin/biotin and avidin/biotin. Examples of suitable fluorescent materials include umbelliferone, fluorescein, fluorescein isothiocyanate, rhodamine, dichlorotriazinylamine fluorescein, dansyl chloride or phycoerythrin. An example of a luminescent material includes luminol. Examples of suitable radioactive material include ¹²⁵I, ¹³¹I, ³⁵S or ³H.

The methods comprise comparison of GPBP-1 levels detected in a test plasma sample with a control, such as a control from a plasma sample known to have "normal" levels of GPBP-1 or previously determined normal values for GPBP-1 in plasma from the subject from whom the plasma is obtained. In various embodiments, the control provides a standard curve using recombinant GPBP-1 or a reference value. In comparing the amount of GPBP-1 in the plasma sample to a control, an increase in GPBP-1 in the plasma sample relative to the control indicates the presence of a pre-diabetic state and/or a propensity to develop T2D.

In one embodiment, the method comprises detecting native circulating GPBP-1 from human plasma. In another embodiment, the GPBP-1 binding molecule comprises an antibody, such as a monoclonal or polyclonal antibody.

### EXAMPLES

GPBP forms large multimeric aggregates whose specific kinase activity is much higher than that of aggregates of lower molecular weight (WO 2000/50607). A five-residue-long motif ²⁶⁰SHCIE²⁶⁴ (SEQ ID NO: 4) is the core of a region which is critical stabilizing native GPBP multimer assembly. An isolated peptide (Q2) representing ²⁶⁰SHCIE²⁶⁴ (SEQ ID NO: 4) and flanking regions (LATLSHCIELMVKR) (SEQ ID NO: 8) efficiently inhibited GPBP kinase activity (WO 2004/070025). Organic compounds mimicking Q2 have been shown to inhibit GPBP kinase activity (WO 2011/054530 and US Patent No: 8,586.776). The anti-tumor agent doxorubicin has also been shown to inhibit GPBP kinase activity (WO2014/006020). Anti-GPBP monoclonal antibodies have also been shown to inhibit GPBP activity (WO 2012/113785).

T2D is associated with insulin resistance and β-cell failure. Insulin and IGF-I exert their biological activities via the phosphorylation and activation of insulin receptor substrate (IRS) proteins (Burks and White, 2001). Deletion of *Irs2* in mice produces peripheral insulin resistance and a reduced in β-cell mass, leading to diabetes (Withers *et al.,* 1998). Male *Irs2*-null mice develop hyperglycemia around 8 weeks of age and most die of diabetic complications before 16 weeks of age. The absence of IRS2 also causes moderate obesity in female mice (Burks *et al.,* 2000). The diabetic phenotype displays a sexual dimorphism such that females develop diabetes more slowly, with many surviving up to 6 months of age (Garcia-Barrado *et al.,* 2011).

### EXPERIMENTAL METHODS

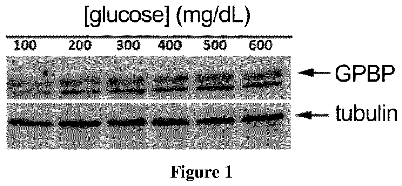

**Figure 1****. Elevated extracellular glucose levels induces GPBP expression.** C2C12 cells were differentiated for 5 days in DMEM (Dulbecco's modified Eagle's medium) supplemented with 2% horse serum and antibiotics. Cultures were washed with PBS (phosphate buffered saline) and incubated for another 24-hour period with DMEM supplemented with 2% horse serum containing glucose at the indicated concentrations. Cells were washed with PBS and lysed in 50 mM Tris-HCl pH 7.0, 150 mM NaCl, 1% Triton X-100, 1 mM PMSF (phenyl-methylsulfonyl fluoride), 10 mg/mL leupeptin for 30 min at 4 °C. Lysates were cleared by centrifugation (16 000 x g, 5 min, 4 °C), the total protein concentration of the supernatants was determined (Bio-Rad) and similar quantities (50 µg) of each extract were analyzed by Western blot with anti-GPBP monoclonal antibodies (mAb) N27 and anti-tubulin (Sigma), as elsewhere indicated.

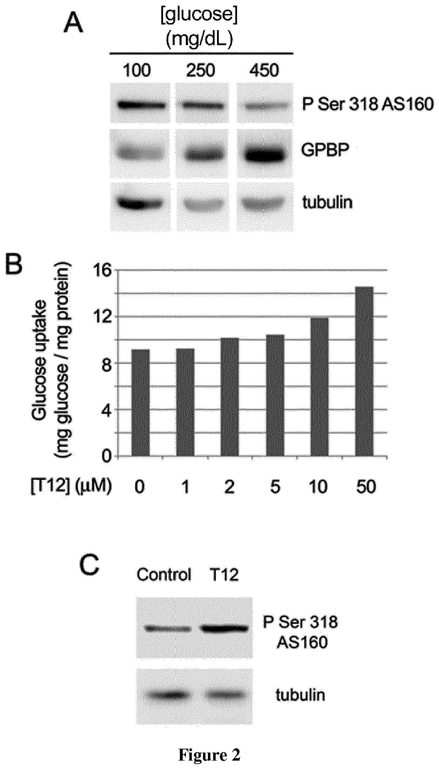

**Figure 2****. GPBP kinase inhibition enhances glucose consumption under increased extracellular glucose concentrations. A,** A549 cells were cultured in DMEM plus 10% fetal bovine serum and penicillin/streptomycin with the indicated concentrations of glucose during 24 h. Then cells were lysed in 25 mM Tris-HCl pH 7.5, 150 mM NaCl, 1% Triton X-100, 0.1% SDS, 1 mM PMSF, 10 µg/mL leupeptin, 20 mM NaF at 4 °C during 30 min. Lysates were cleared by centrifugation at 16,000 x g for 5 min (4 °C) and the protein concentration of supernatants was determined (Bio-Rad). Similar amounts (50 µg) of each lysate were analyzed by Western blot for detection of AS160 protein phosphorylated at Ser 318 (P Ser 318 AS160), GPBP and tubulin, using primary antibodies obtained from Cell Signaling Technology, Fibrostatin SL and Sigma, respectively, and HRP-conjugated anti-rabbit IgG and anti-mouse IgG secondary antibodies from Promega. Development was performed with Amersham ECL Prime Western Blotting Detection Reagent (GE Healthcare Lifesciences) and image acquisition with an ImageQuant LAS 4000 Mini device (GE Healthcare Lifesciences). **B,** A549 cells were cultured in DMEM: F-12 (315 mg glucose/dL) supplemented with 10% fetal bovine serum and penicillin/streptomycin with the indicated concentrations of T12 during 14 h. After incubations cells were lysed as in **A** and protein concentrations in supernatants were similarly assessed. Glucose concentrations in the media were determined at the beginning and at the end of the incubation period with a Glucocard G+ meter (Arkray Factory, Inc.), and glucose uptake by cells was calculated using the protein concentrations of the corresponding lysates for normalization purposes. Similar results were obtained with doxorubicin (0.5 µM) (data not shown), a different GPBP kinase inhibitor (WO/2014/006020). C, A549 cells were incubated in DMEM: F-12 (315 mg glucose/dL) plus 10% fetal bovine serum and penicillin/streptomycin during 20 h in absence (Control) or presence of T12 (50 µM), and lysed as in **A**. Similar amounts (50 µg) of each lysate were analyzed by Western blot for detection of AS160 protein phosphorylated at Ser 318 (P Ser 318 AS160) or of tubulin as in **A**.

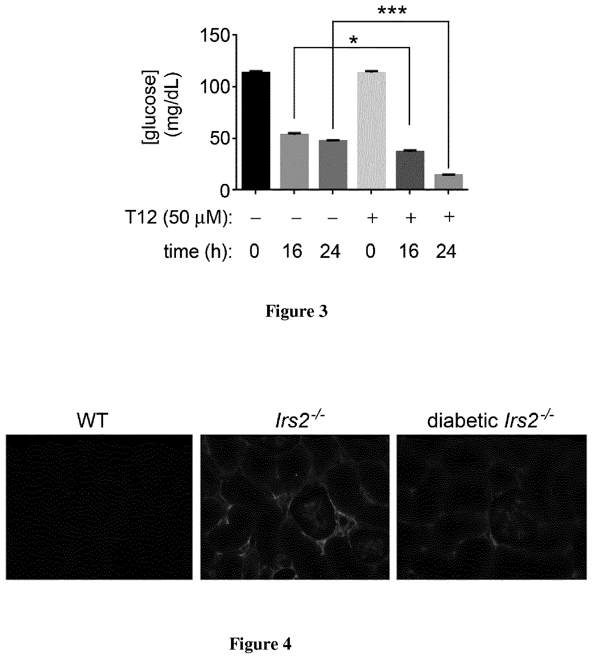

**Figure 3****. GPBP kinase inhibition also enhances glucose consumption under physiological extracellular glucose concentrations.** C2C12 mouse myoblasts were differentiated to myotubes in DMEM supplemented with 2% horse serum and penicillin/streptomycin during 10 days, and then incubated in DMEM plus 0.25% horse serum with an initial glucose concentration of 115 mg/dL, in absence or presence of T12 (50 µM) during the indicated times. Glucose concentrations in media were determined with a Glucocard™ G+ meter (Arkray Factory, Inc.). Shown are means ± SEM (n=2). Differences between the indicated groups were statistically significant according to t-Student test analysis. *P=0.0152; ***P=0.0005.
**Figure 4****. *Irs2*^{*-*/*-*} mice displayed increased collagen IV expression.** Kidneys were collected from male wild type (WT, n=9), normoglycemic *Irs2*^{*-*/*-*} (*Irs2^{-l-},* n=9) and diabetic *Irs2*^{*-*/*-*} (n=9) mice at 8-12 weeks of age and embedded in paraffin for immunohistochemical analysis of sections with anti-type IV collagen antibodies from EMD Millipore. Mice were considered diabetic when serum glucose levels under fasting were greater than 120 mg/dL. In the composite the level of collagen IV expression is represented in a black (no expression) and white (expression) color range. Shown are representative images. Original magnification: x 400.

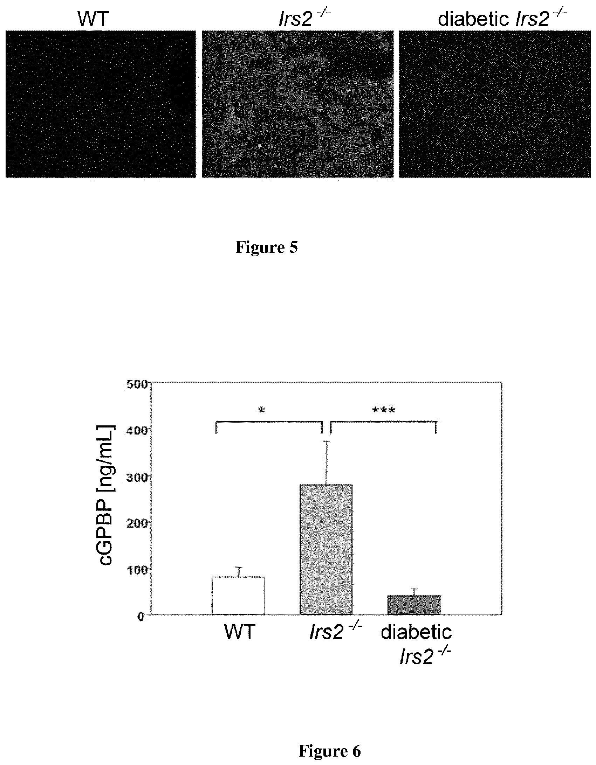

**Figure 5****. *Irs2*^{*-*/*-*} mice displayed increased *COL4A3BP* expression.** Kidneys were collected from male wild type (WT, n=9), normoglycemic *Irs2*^{*-*/*-*} (*Irs2*^{*-*/}*⁻,* n=9) and diabetic *Irs2*^{*-*/*-*} (n=9) mice at 8-12 weeks of age and embedded in paraffin for immunohistochemical analysis of sections with anti-GPBP mAb N26 from Fibrostatin SL. Mice were considered diabetic when serum glucose levels under fasting were greater than 120 mg/dL. In the composite the level of GPBP expression is represented in a black (no expression) and white (expression) color range. Shown are representative images. Original magnification: x 400.
**Figure 6****. Circulating GPBP levels are augmented in normoglycemic Irs2-deficient mice.** Blood samples were collected from wild type (WT, n=12), normoglycemic *Irs2*^{*-*/*-*} (*Irs2*^{*-*/}*⁻,* n=12) and diabetic *Irs2*^{*-*/*-*}(n=19) mice at the time of sacrifice (8-12 weeks of age) and circulating GPBP levels were measured using mAb N26 as capturing and mAb N27 as detecting antibodies in a prototype sandwich ELISA essentially as described in WO 2010/009856. Mice were considered diabetic when serum glucose levels under fasting were greater than 120 mg/dL. *P<0.05, ***P <0.001.

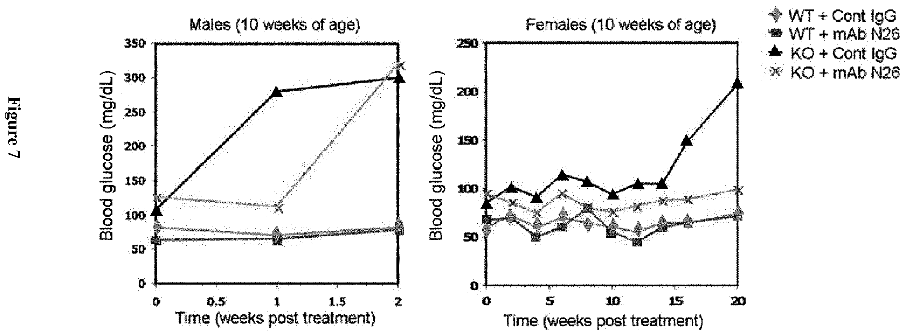

**Figure 7****. Effect of anti-GPBP treatment on blood glucose in 10-week old mice.** Different groups of mice received weekly intraperitoneal injections of anti-GPBP mAb N26 or mouse IgG (Sigma) at 1 µg/g of body weight, and fasting blood glucose (tail vein) was monitored weekly using a glucometer. *Males.* Wild type treated (WT + mAb N26, n=2), wild type control (WT + Cont IgG, n=2), *Irs2*^{*-*/*-*} treated (KO + mAb 26, n=2), *Irs2*^{*-*/*-*} control (KO + Cont IgG, n=2). *Females.* Wild type treated (n=2), wild type control (n=2), *Irs2*^{*-*/*-*} treated (n=3), *Irs2*^{*-*/*-*}control (n=2).

### RESULTS AND DISCUSSION

In differentiated myoblasts, downregulation of GPBP expression (i.e. *Gpbp*^{*-*/*-*}) associated with increased activation of Akt (Revert-Ros et *al.,* 2011) revealing that cytosolic GPBP downregulates Akt activation and renders the cells with less capacity to uptake glucose in response to insulin (insulin resistance). Consistently, increasing extracellular glucose induced GPBP expression in differientated myoblast **(****Fig. 1****)** suggesting that intracellular levels of GPBP increase in response to increasing extracellular glucose concentrations (hyperglycaemia) perhaps in an attempt of the cells to prevent deleterious effects of elevated intracellular glucose concentrations. Accordingly, when exposing A549 cultures to increasing concentration of extracellular glucose we observed a progressive increased expression of GPBP which associated with a lower phosphorylation state of Akt-substrate 160 (AS160), the hallmark for induction of glucose uptake **(****Fig. 2A****).** Moreover, increasing concentrations of GPBP kinase inhibitor compound T12 (3-[4"-methoxy-3,2'-dimethyl-(1,1;4',1")terphenyl-2"-yl]propionic acid (WO 2011/054530 and US Patent No: 8,586.776); progressively enhanced glucose uptake in A549 cells cultured under conditions representing the hyperglycaemic state of T2D **(****Fig. 2B****).** All of which suggested that cytosolic GPBP kinase inhibition by compound T12 induced glucose uptake through Akt disinhibition and subsequent phosphorylation of AS160. Accordingly, analysis of the corresponding cell cultures lysates by Western blot revealed that compound T12 induced phosphorylation of AS160 **(****Fig. 2C****)** which in turn is expected to promote translocation of glucose transporters GLUT1/GLUT4 to the plasma membrane and glucose uptake (Sano *et al.,* 2003). Collectively, the evidence suggests that enhanced cytosolic kinase activity of GPBP associated with hyperglycemia mediates at least in part peripheral insulin resistance. Similar conclusions were obtained when treating cell cultures with doxorubicin (0.5 µM) (data not shown), an additional structurally unrelated GPBP kinase inhibitor (WO2014/006020). Further, the role of GPBP kinase activity was assessed in physiological glucose concentrations and shown that compound T12 also reduced the extracellular glucose levels in a concentration-dependent manner **(****Fig. 3****),** suggesting that cytosolic GPBP kinase activity down regulates insulin-dependent signaling in both normo-glycemic and diabetic conditions.

To investigate the role of GPBP in T2D we have first analyzed the expression of both GPBP and collagen IV in the kidneys of *Irs2*^{*-*/*-*} mice. Diabetic nephropathy is characterized by accumulation of mesangial matrix and thickening of the basement membrane in the glomeruli as well as renal tubular hypertrophy. These abnormalities are associated with renal overproduction of extracellular matrix proteins such as collagen IV. Consistent with this, clinical studies have reported a progressive increase in urinary collagen IV excretion in diabetic patients (Cawood *et al.,* 2010). Consequently, a high level of urinary collagen IV has been proposed as a marker of development and progression of early diabetic kidney disease. In assessing diabetes in the *Irs2*^{*-*/*-*} mouse model, we apply the same criteria for blood glucose values as in humans; fasting levels > 120 mg/dL are considered diabetic. We observed that mice with severe hyperglycemia often presented proteinuria (>100 mg/dL), suggesting a deterioration of renal function associated with high glucose levels. Thus, we used immunohistochemistry to assess collagen IV expression in kidneys obtained from wild type (WT) *Irs2* ^{+/+} mice, normoglycemic *Irs2* ^{-/-} mice and diabetic *Irs2* ^{-/-} mice. We observed that the expression of collagen IV was increased in both groups of *Irs2* ^{-/-} mice in comparison to WT control kidney but being more expressed in normoglycemic than in diabetic *Irs2* ^{-/-} mice **(****Fig. 4****).** Given the role of GPBP in regulating collagen IV supramolecular organization and triggering glomerulosclerosis when overexpressed (Revert *et al.,* 2007), we examined GPBP expression in kidney sections of control and *Irs2*^{*-*/*-*} mice. Interestingly, GPBP expression paralleled collagen IV expression and thus GPBP was overexpressed in the kidneys of *Irs2*^{*-*/*-*} mice as compared with WT controls and still more expressed in normoglycemic than in diabetic *Irs2* ^{-/-} mice **(****Fig. 5****).** Thus, these findings suggest that pro-inflammatory cytokines associated with the pre-diabetic state in *Irs2*^{*-*/*-*} mice may up regulate GPBP expression which then declines to a more basal level in mice with overt diabetes (inflammatory stage). These results are consistent with GPBP being a pre-pro-inflammatory circulating factor (WO 2012/113785) that also plays a role in T2D of *Irs2*^{*-*/*-*} mouse model.

To further assess this possibility we used a previously developed ELISA for detection of circulating GPBP (WO 2010/009856 and US Patent No: 7,935.492) to measure the levels of GPBP in serum samples obtained from control mice and the two groups of *Irs2*^{*-*/*-*} (normoglycemic and diabetic). Consistent with the results of the immunostainings, circulating levels of GPBP were significantly increased in normoglycemic *Irs2*^{*-*/*-*}mice as compared with WT **(****Fig. 6****).** However, in diabetic *Irs2*^{*-*/*-*} mice, we observed a marked reduction in circulating levels of GPBP. These results suggest that up regulation of GPBP occurs in the pre-diabetic state likely in parallel with pro-inflammatory cytokines. Hence, given the availability of a sensitive ELISA for detecting GPBP in serum, our observations in the *Irs2*^{*-*/*-*} model indicate that GPBP is a potential novel biomarker of the pre-diabetic state that could be used clinically for the early detection of insulin resistance and diabetes.

Deletion of *Irs2* produces diabetes in mice owing to peripheral insulin resistance and a reduction in pancreatic β-cell mass (Withers *et al.,* 1998). However, this diabetic phenotype displays a sexual dimorphism; male *Irs2*^{*-*/*-*} mice often die of diabetic complications by 12 weeks of age but female *Irs2*^{*-*/*-*} develop a milder form of diabetes and many live up to 6 months (Garcia-Barrado, 2011). Additionally, female *Irs2*^{*-*/*-*} mice display hyperleptinemia and develop moderate obesity, in contrast to male *Irs2*^{*-*/*-*} which are often leaner than control mice (Burks *et al.,* 2000).

Our observation that GPBP and collagen IV are up regulated in normoglycemic *Irs2*^{*-*/*-*} mice suggests that this kinase may be involved in the progression from the pre-diabetic to the diabetic state. If indeed GPBP has a role in this pathology, GPBP inhibition would be expected to delay or prevent development of diabetes in pre-diabetic models. To test this possibility, normoglycemic *Irs2*^{*-*/*-*} mice were treated with N26, GPBP-specific antibodies which inhibit the binding of GPBP to the α3NC1 domain of collagen IV (WO 2012/113785). Glucose levels were then monitored as an indicator of metabolic status. Given the sexual dimorphism present in this model of diabetes, we assessed anti-GPBP in both males and females. In this study, treatment was begun when mice were 10 weeks of age and N26 was administered at 1 µg/g of body weight once weekly via an intraperitoneal injection. Fasting glucose levels in WT females and males treated with anti-GPBP were similar to those treated with a control IgG, demonstrating that the anti-GPBP treatment did not alter basal glucose levels **(****Fig. 7****).** Although treatment was able to maintain glycaemia at normal levels in *Irs2*^{*-*/*-*} males a week after treatment initiation, hyperglycemia proceeded rapidly during the second week and reached similar values regardless of whether mice received injections of N26 or not; and thus all *Irs2*^{*-*/*-*} males died of diabetic complications within a month of the study onset. In contrast, female *Irs2*^{*-*/*-*} treated with anti-GPBP therapy lived for up to 7 months with normal glucose levels whereas the female *Irs2*^{*-*/*-*} treated with a control IgG progressively developed hyperglycaemia.

Collectively, the data support that pre-pro-inflammatory extracellular GPBP (WO 2012/113785) mediates pathogenesis in the *Irs2*^{*-*/*-*} model for T2D. This also supports the notion that increased circulating levels of GPBP are diagnostic of incoming inflammation that mediates hyperglycaemia and T2D. Accordingly, early administration of GPBP blocking antibodies (pre-diabetic) delayed hyperglycaemia onset in males and precluded hyperglycaemia in females. Thus, we uncovered extracellular GPBP to be a new potential biomarker for detecting pre-diabetic stage as well as a new potential therapeutic target for treating T2D. Moreover, anti-GPBP antibodies emerge as a double-edge sword against T2D detecting patients that will develop T2D and as a biological drug to treat these patients.

Of special interest was to observe that individual administration of three unrelated down regulators of GPBP (i.e. T12, doxorubicin and N26) in different experimental models (i.e. myocyte, lung adenocarcinoma A549 cell line and *Irs2*^{*-*/*-*} mouse) reduced extracellular glucose concentrations. The evidence also supports that reduction of extracellular glucose is accomplished at least in part through phosphorylation of the cytosolic protein AS160 leading to enhanced glucose uptake. This observation provides further independent evidence for extracellular GPBP to regulate intracellular GPBP activity mediating pathogenesis in the *Irs2*^{*-*/*-*} mouse model for T2D.

### REFERENCES

Burks DJ, Font de Mora J, Schubert M, Withers DJ, Myers MJ, Towery H, Altamuro SA, Flint CA, White MF. 2000. IRS-2 pathways integrate female reproduction and energy homeostasis. Nature 407:377-82.
Burks DJ, White MF. 2001. The role of IRS proteins in beta cell physiology. Diabetes 456: 120-125.
Cawood TJ, Bashir M, Brady J, Murray B, Murray PT, O'Shea D. 2010. Urinary collagen IV and πGST: potential biomarkers for detecting localized kidney injury in diabetes--a pilot study. Am. J. Nephrol. 32:219-25.
Garcia-Barrado MJ, Iglesias-Osma MC, Moreno-Viedma V, Pastor Mansilla MF, Gonzalez SS, Carretero J, Moratinos J, Burks DJ. 2011. Differential Sensitivity To Adrenergic Stimulation Underlies The Sexual Dimorphism In The Development Of Diabetes Caused By Irs-2 Deficiency. Biochem Pharmacol. 81:279-88.
Revert F, Merino R, Monteagudo C, Macias J, Peydró A, Alcácer J, Muniesa P, Marquina R, Blanco M, Iglesias M, Revert-Ros F, Merino J, Saus J. 2007. Increased Goodpasture antigen-binding protein expression induces type IV collagen disorganization and deposit of immunoglobulin A in glomerular basement membrane. Am. J. Pathol. 171:1419-30.
Revert-Ros F, López-Pascual E, Granero-Moltó F, Macías J, Breyer R, Zent R, Hudson BG, Saadeddin A, Revert F, Blasco R, Navarro C, Burks D, Saus J. Goodpasture antigen-binding protein (GPBP) directs myofibril formation: identification of intracellular downstream effector 130-kDa GPBP-interacting protein (GIP130). J Biol Chem. 286:35030-43.
Sano H, Kane S, Sano E, Mîinea CP, Asara JM, Lane WS, Garner CW, Lienhard GE. 2003. Insulin-stimulated phosphorylation of a Rab GTPase-activating protein regulates GLUT4 translocation. J Biol Chem. 278:14599-602.
Withers DJ, Gutierrez JS, Towery H, Burks DJ, Ren JM, Previs S, Zhang Y, Bernal D, Pons S, Shulman GI, Bonner-Weir S, White MF. 1998. Disruption of IRS-2 causes type 2 diabetes in mice. Nature. 391:900-4.

## Claims

1. A Goodpasture Antigen Binding Protein-1 (GPBP-1) inhibitor for use in the treatment of the pre-diabetic state.

2. The GPBP-1 inhibitor for use of claim 1, wherein the subject has increased circulating GPBP-1 relative to control, wherein control is a control sample from a normal subject or previously determined normal levels of GPBP-1 in a control population.

3. The GPBP-1 inhibitor for use of any one of claims 1-2, wherein the GPBP-1 inhibitor comprises an anti-GPBP-1 monoclonal antibody, such as a humanized monoclonal antibody.

4. The GPBP-1 inhibitor for use of any one of claims 1-3, wherein the GPBP-1 inhibitor comprises a compound of formula: 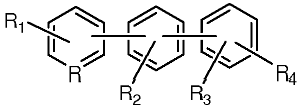 or a pharmaceutically acceptable salt thereof, wherein:
R is selected from N and CR₅; R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C₆ alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), -(CH₂)₁₋₅-C(O)NH₂, (aryl)C₂-C₆ alkyl, and (heteroaryl)C₁-C₆ alkyl;
R₁ is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
R₂ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, (aryl)C₁-C₆ alkyl, or (heteroaryl)C₁-C₆ alkyl;
R₃ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₁-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), (aryl)C₁-C₆ alkyl, or (heteroaryl)C₁-C₆ alkyl; and
R₄ is hydroxy, halogen, C₁-C₆ alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -O(CH₂)₁₋₅-C(O)OH, -O(CH₂)₁₋₅-C(O)(C₁-C₆ alkoxy), (aryl)C₁-C₆ alkyl, or (heteroaryl)C₁-C₆ alkyl.

5. The GPBP-1 inhibitor for use of any one of claims 1-4, wherein the GPBP-1 inhibitor comprises a compound having the formula: 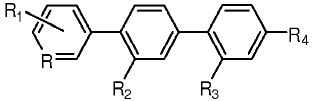 or a pharmaceutically acceptable salt thereof, wherein:
R is selected from N and CR₅;
R₅ is selected from the group consisting of hydrogen, halogen, cyano, nitro, hydroxy, C₁-C₆ alkyl, C₂-C₆ alkenyl, C₂-C₆ alkynyl, halo(C₁-C₆ alkyl), C₁-C₆ alkoxy, halo(C₁-C6 alkoxy), amino, (C₁-C₆ alkyl)amino, di(C₁-C₆ alkyl)amino, hydroxy(C₁-C₆ alkyl), (C₁-C₆ alkoxy)C₁-C₆ alkyl, amino(C₁-C₆ alkyl), sulfanyl(C₁-C₆ alkyl), (C₁-C₆ alkyl)sulfanyl(C₁-C₆ alkyl), -(CH₂)₁₋₅-C(O)(C₁-C₆ alkoxy), and -(CH₂)₁₋₅-C(O)NH₂;
R₁ is hydrogen, halogen, hydroxy, C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, amino(C₁-C₆ alkyl), sulfanyl(C₁-C₆ alkyl), or (C₁-C₆ alkyl)sulfanyl(C₁-C₆ alkyl);
R₂ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₀-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), or -(CH₂)₁₋₅-C(O)NH₂;
R₃ is C₁-C₆ alkyl, halo(C₁-C₆ alkyl), C₁-C₆ alkoxy, halo(C₁-C₆ alkoxy), hydroxy(C₁-C₆ alkyl), (C₁-C₆ alkoxy)C₁-C₆ alkyl, formyl(C₁-C₆ alkyl), amino(C₁-C₆ alkyl), sulfanyl(C₁-C₆ alkyl), (C₁-C₆ alkyl)sulfanyl(C₁-C₆ alkyl), -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, or -CH=CH-C(O)(C₁-C₆ alkoxy); and
R₄ is hydroxy, halogen, C₁-C₆ alkyl, C₁-C₆ alkoxy, halo(C₁-C₆ alkoxy), benzyloxy, -(CH₂)₁₋₅-C(O)OH, -(CH₂)₁₋₅-C(O)(C₁-C₆ alkoxy), -(CH₂)₁₋₅-C(O)NH₂, -(CH₂)₁₋₅-C(O)NH(C₁-C₆ alkyl), -(CH₂)₁₋₅-C(O)N(C₁-C₆ alkyl)₂, -CH=CH-C(O)OH, -CH=CH-C(O)(C₁-C₆ alkoxy), -O(CH₂)₁₋₅-C(O)OH, or -O(CH₂)₁₋₅-C(O)(C₁-C₆ alkoxy).

6. The GPBP-1 inhibitor for use of any one of claims 1-5, wherein the GPBP-1 inhibitor comprises 3-[4"-methoxy-3,2'-dimethyl-(1,1';4',1")terphenyl-2"-yl]propionic acid; 3-[2'-methyl-4"-methoxy-3-(trifluoromethyl)-(1,1';4',1")terphenyl-2"-yl]propionic acid; combinations thereof, or a pharmaceutically acceptable salt thereof.

7. The GPBP-1 inhibitor for use of any one of claims 1-6, wherein the GPBP-1 inhibitor comprises (3-[4"-methoxy-3,2'-dimethyl-(1,1';4',1")terphenyl-2"-yl]propionic acid, or a pharmaceutically acceptable salt thereof.

8. The GPBP-1 inhibitor for use of any one of claims 1-7, wherein the GPBP-1 inhibitor comprises one or more inhibitors selected from the group consisting of:
(a) an antibody or aptamer selective for GPBP-1;
(c) an inhibitory nucleic acid selective for GPBP-1 mRNA;
(d) a peptide comprising an amino acid sequence of general formula X1-SHCIX2-X3 (SEQ ID NO: 1)
wherein X1 is 0-10 amino acids of the sequence ATTAGILATL (SEQ ID NO: 2);
X2 is E or Q; and
X3 is 0-10 amino acids of the sequence LMVKREDSWQ (SEQ ID NO: 3);
(e) a peptide comprising an amino acid sequence selected from the group consisting of SHCIE (SEQ ID NO: 4), SHCIQ (SEQ ID NO: 5), ILATLSHCIELMVKR (SEQ ID NO: 6), ILATLSHCIQLMVKR (SEQ ID NO: 7), and LATLSHCIELMVKR (SEQ ID NO: 8); and
(f) I-20:

9. A method for diagnosing a pre-diabetic state comprising
(a) determining an amount of GPBP-1 in a sample from a subject at risk of having a pre-diabetic state; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample indicates the presence of a pre-diabetic state in the subject.

10. A method for diagnosing a propensity to develop type 2 diabetes, comprising
(a) determining an amount of GPBP-1 in a sample from a subject at risk of developing type 2 diabetes; and
(b) comparing the amount of GPBP-1 in the sample to an amount of GPBP-1 in a control sample, wherein the control sample is a sample from a normal subject;
wherein an increase in the amount of GPBP-1 in the sample compared to the control sample indicates a propensity to develop type 2 diabetes in the subject.

11. The method of claim 9 or 10, wherein the diagnosing comprises
(a) contacting a plasma sample from the subject with a GPBP-1 binding molecule that binds to GPBP-1 under conditions to promote selective binding of the GPBP-1 binding molecule to the GPBP-1;
(b) removing unbound plasma and/or GPBP-1 binding molecules; and
(c) detecting complex formation between the GPBP-1 binding molecule and the GPBP-1 in the plasma sample, wherein complex formation provides a measure of GPBP-1 in the sample.

12. The method of any one of claims 9-11, wherein the GPBP-1 binding molecule comprises an antibody, such as a monoclonal antibody.

## Patentansprüche

1. Inhibitor von Goodpasture-Antigen-Bindungsprotein-1 (GPBP-1) zur Verwendung bei der Behandlung des prädiabetischen Zustands.

2. Inhibitor von GPBP-1 zur Verwendung nach Anspruch 1, wobei das Subjekt im Vergleich zur Kontrolle erhöhte Werte an zirkulierendem GPBP-1 aufweist, wobei die Kontrolle eine Kontrollprobe aus einem normalen Subjekt ist oder zuvor bestimmte normale Level von GPBP-1 in einer Kontrollpopulation sind.

3. Inhibitor von GPBP-1 zur Verwendung nach einem der Ansprüche 1 oder 2, wobei der Inhibitor von GPBP-1 ein monoklonaler anti-GPBP-1-Antikörper, wie zum Beispiel ein humanisierter monoklonaler Antikörper ist.

4. Inhibitor von GPBP-1 zur Verwendung nach einem der Ansprüche 1 bis 3, wobei der Inhibitor von GPBP-1 eine Verbindung der Formel 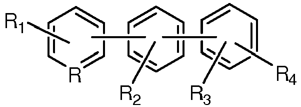 oder ein pharmazeutisch verträgliches Salz derselben umfasst, wobei:
R ausgewählt ist aus N und CR₅;
R₅ ausgewählt ist aus der Gruppe bestehend aus
Wasserstoff, Halogen, Cyano, Nitro, Hydroxy, C₁-C₆-Alkyl, C₂-C₆-Alkenyl, C₂-C₆-Alkinyl, Halo(C₁-C₆-alkyl), C₁-C₆-Alkoxy, Halo(C₁-C₆-alkoxy), Amino, (C₁-C₆-Alkyl)amino, Di(C₁-C₆-alkyl)amino, Hydroxy(C₁-C₆-alkyl), (C₁-C₆-Alkoxy)C₁-C₆-alkyl, Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)(C₁-C₆-Alkoxy), -(CH₂)₁₋₅-C(O)NH₂, (Aryl)C₂-C₆-alkyl und (Heteroaryl)C₁-C₆-alkyl;
R₁ Wasserstoff, Halogen, Hydroxy, C₁-C₆-Alkyl, Halo(C₁-C₆-alkyl), C₁-C₆-Alkoxy, Halo(C₁-C6-alkoxy), Hydroxy(C₁-C₆-alkyl), (C₁-C₆-Alkoxy)C₁-C₆-alkyl, Amino(C₁-C₆-alkyl), Sulfanyl(C₁-C₆-alkyl) oder (C₁-C₆-Alkyl)sulfanyl(C₁-C₆-alkyl) ist;
R₂ C₁-C₆-Alkyl, Halo(C₁-C₆-alkyl), C₁-C₆-Alkoxy, Halo(C₁-C₆-alkoxy), Hydroxy(C₁-C₆-alkyl), (C₁-C₆-Alkoxy)C₁-C₆-alkyl, Formyl(C₀-C₆-alkyl), Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)OH, -(CH₂)₁₋₅-C(O)(C₁-C₆-Alkoxy), -(CH₂)₁₋₅-C(O)NH₂, (Aryl)C₁-C₆-alkyl oder (Heteroaryl)C₁-C₆-alkyl ist;
R₃ C₁-C₆-Alkyl, Halo(C₁-C₆-alkyl), C₁-C₆-Alkoxy, Halo(C₁-C₆-alkoxy), Hydroxy(C₁-C₆-alkyl), (C₁-C₆-Alkoxy)C₁-C₆-alkyl, Formyl(C₁-C₆-alkyl), Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)OH, -(CH₂)₁₋₅-C(O)(C₁-C₆-Alkoxy), -(CH₂)₁₋₅-C(O)NH₂, -(CH₂)₁₋₅-C(O)NH(C₁-C₆-Alkyl), -(CH₂)₁₋₅-C(O)N(C₁-C₆-Alkyl)₂, -CH=CH-C(O)OH, -CH=CH-C(O)(C₁-C₆-Alkoxy), (Aryl)C₁-C₆-alkyl oder (Heteroaryl)C₁-C₆-alkyl ist; und
R₄ Hydroxy, Halogen, C₁-C₆-Alkyl, C₁-C₆-Alkoxy, Halo(C₁-C₆-alkoxy), Benzyloxy, -(CH₂)₁₋₅-C(O)OH, -(CH₂)₁₋₅-C(O)(C₁-C₆-Alkoxy), -(CH₂)₁₋₅-C(O)NH₂, -(CH₂)₁₋₅-C(O)NH(C₁-C₆-Alkyl), -(CH₂)₁₋₅-C(O)N(C₁-C₆-Alkyl)₂, -CH=CH-C(O)OH, -CH=CH-C(O)(C₁-C₆-Alkoxy), -O(CH₂)₁₋₅-C(O)OH, -O(CH₂)₁₋₅-C(O)(C₁-C₆-Alkoxy), (Aryl)C₁-C₆-alkyl oder (Heteroaryl)C₁-C₆-alkyl ist.

5. Inhibitor von GPBP-1 zur Verwendung nach einem der Ansprüche 1 bis 4, wobei der Inhibitor von GPBP-1 eine Verbindung mit der Formel 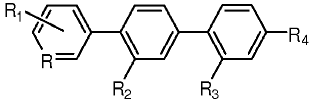 oder ein pharmazeutisch verträgliches Salz derselben umfasst, wobei:
R ausgewählt ist aus N und CR₅;
R₅ ausgewählt ist aus der Gruppe bestehend aus Wasserstoff, Halogen, Cyano, Nitro, Hydroxy, C₁-C₆-Alkyl, C₂-C₆-Alkenyl, C₂-C₆-Alkinyl, Halo(C₁-C₆-alkyl), C₁-C₆-Alkoxy, Halo(C₁-C₆-alkoxy), Amino, (C₁-C₆-Alkyl)amino, Di(C₁-C₆-alkyl)amino, Hydroxy(C₁-C₆-alkyl), (C₁-C₆-Alkoxy)C₁-C₆-alkyl, Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)(C₁-C₆-Alkoxy) und -(CH₂)₁₋₅-C(O)NH₂;
R₁ Wasserstoff, Halogen, Hydroxy, C₁-C₆-Alkyl, Halo(C₁-C₆-alkyl), C₁-C₆-Alkoxy, Halo(C₁-C₆-alkoxy), Hydroxy(C₁-C₆-alkyl), (C₁-C₆-Alkoxy)C₁-C₆-alkyl, Amino(C₁-C₆-alkyl), Sulfanyl(C₁-C₆-alkyl) oder (C₁-C₆-Alkyl)sulfanyl(C₁-C₆-alkyl) ist;
R₂ C₁-C₆-Alkyl, Halo(C₁-C₆-alkyl), C₁-C₆-Alkoxy, Halo(C₁-C₆-alkoxy), Hydroxy(C₁-C₆-alkyl), (C₁-C₆-Alkoxy)C₁-C₆-alkyl, Formyl(C₀-C₆-alkyl), Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)OH, -(CH₂)₁₋₅-C(O)(C₁-C₆-Alkoxy) oder -(CH₂)₁₋₅-C(O)NH₂ ist;
R₃ C₁-C₆-Alkyl, Halo(C₁-C₆-alkyl), C₁-C₆-Alkoxy, Halo(C₁-C₆-alkoxy), Hydroxy(C₁-C₆-alkyl), (C₁-C₆-Alkoxy)C₁-C₆-alkyl, Formyl(C₁-C₆-alkyl), Amino(C₁-C₆-alkyl), Sulfanyl(C₁-C₆-alkyl), (C₁-C₆-Alkyl)sulfanyl(C₁-C₆-alkyl), -(CH₂)₁₋₅-C(O)OH, -(CH₂)₁₋₅-C(O)(C₁-C₆-Alkoxy), -(CH₂)₁₋₅-C(O)NH₂, -(CH₂)₁₋₅-C(O)NH(C₁-C₆-Alkyl), -(CH₂)₁₋₅-C(O)N(C₁-C₆-Alkyl)₂, -CH=CH-C(O)OH oder -CH=CH-C(O)(C₁-C₆-Alkoxy) ist; und
R₄ Hydroxy, Halogen, C₁-C₆-Alkyl, C₁-C₆-Alkoxy, Halo(C₁-C₆-alkoxy), benzyloxy, -(CH₂)₁₋₅-C(O)OH, -(CH₂)₁₋₅-C(O)(C₁-C₆-Alkoxy), -(CH₂)₁-₅-C(O)NH₂, -(CH₂)₁₋₅-C(O)NH(C₁-C₆-Alkyl), -(CH₂)₁₋₅-C(O)N(C₁-C₆-Alkyl)₂, -CH=CH-C(O)OH, -CH=CH-C(O)(C₁-C₆-Alkoxy), -O(CH₂)₁₋₅-C(O)OH oder -O(CH₂)₁₋₅-C(O)(C₁-C₆-Alkoxy) ist.

6. Inhibitor von GPBP-1 zur Verwendung nach einem der Ansprüche 1 bis 5, wobei der Inhibitor von GPBP-1 3-[4"-Methoxy-3,2'-dimethyl-(1,1';4',1")terphenyl-2"-yl]propionsäure; 3-[2'-Methyl-4"-methoxy-3-(trifluormethyl)-(1,1';4',1")terphenyl-2"-yl]propionsäure; Kombinationen davon oder ein pharmazeutisch verträgliches Salz davon umfasst.

7. Inhibitor von GPBP-1 zur Verwendung nach einem der Ansprüche 1 bis 6, wobei der Inhibitor von GPBP-1 (3-[4"-Methoxy-3,2'-dimethyl-(1,1';4',1")terphenyl-2"-yl]propionsäure oder ein pharmazeutisch verträgliches Salz davon umfasst.

8. Inhibitor von GPBP-1 zur Verwendung nach einem der Ansprüche 1 bis 7, wobei der Inhibitor von GPBP-1 einen oder mehrere Inhibitoren umfasst, die aus der Gruppe ausgewählt sind, die aus
(a) einem für GPBP-1 selektiven Antikörper oder Aptamer;
(c) einer für GPBP-1-mRNA selektiven, inhibitorischen Nukleinsäure;
(d) einem Peptid, welches eine Aminosäuresequenz der allgemeinen Formel X1-SHCIX2-X3 (SEQ ID NO: 1) umfasst,
wobei X1 0-10 Aminosäuren der Sequenz ATTAGILATL (SEQ ID NO: 2) darstellt;
X2 E oder Q ist; und
X3 0-10 Aminosäuren der Sequenz LMVKREDSWQ (SEQ ID NO: 3) darstellt;
(e) einem Peptid, welches eine Aminosäuresequenz umfasst, die aus der Gruppe bestehend aus SHCIE (SEQ ID NO: 4), SHCIQ (SEQ ID NO: 5), ILATLSHCIELMVKR (SEQ ID NO: 6), ILATLSHCIQLMVKR (SEQ ID NO: 7), und LATLSHCIELMVKR (SEQ ID NO: 8) ausgewählt ist; and
(f) I-20: besteht.

9. Verfahren zum Diagnostizieren eines prädiabetischen Zustands, umfassend:
(a) Bestimmen einer Menge an GPBP-1 in einer Probe von einem Subjekt, das ein Risiko für einen prädiabetischen Zustand aufweist; und
(b) Vergleichen der Menge an GPBP-1 in der Probe mit einer Menge an GPBP-1 in einer Kontrollprobe, wobei die Kontrollprobe eine Probe von einem normalen Subjekt ist;
wobei eine Erhöhung in der Menge an GPBP-1 in der Probe im Vergleich zur Kontrollprobe das Vorhandensein eines prädiabetischen Zustands in dem Subjekt anzeigt.

10. Verfahren zum Diagnostizieren einer Neigung, Typ-2-Diabetes zu entwickeln, umfassend:
(a) Bestimmen einer Menge an GPBP-1 in einer Probe von einem Subjekt, das ein Risiko für die Entwicklung von Typ-2-Diabetes aufweist; und
(b) Vergleichen der Menge an GPBP-1 in der Probe mit einer Menge an GPBP-1 in einer Kontrollprobe, wobei die Kontrollprobe eine Probe von einem normalen Subjekt ist;
wobei eine Erhöhung in der Menge an GPBP-1 in der Probe im Vergleich zur Kontrollprobe eine Neigung in dem Subjekt anzeigt, Typ-2-Diabetes zu entwickeln.

11. Verfahren nach Anspruch 9 oder 10, wobei das Diagnostizieren umfasst:
(a) In-Kontakt-Bringen einer Plasmaprobe von dem Subjekt mit einem GPBP-1-Bindungsmolekül, welches an GPBP-1 unter Bedingungen zum Fördern der selektiven Bindung des GPBP-1-Bindungsmoleküls an das GPBP-1 bindet;
(b) Entfernen von ungebundenem Plasma und/oder ungebundenen GPBP-1 - Bindungsmolekülen; und
(c) Detektieren von Komplexbildung zwischen dem GPBP-1-Bindungsmolekül und dem GPBP-1 in der Plasmaprobe, wobei Komplexbildung ein Maß für GPBP-1 in der Probe bietet.

12. Verfahren nach einem der Ansprüche 9-11, wobei das GPBP-1-Bindungsmolekül einen Antikörper, wie zum Beispiel einen monoklonalen Antikörper, umfasst.

## Revendications

1. Inhibiteur de la protéine-1 de liaison à l'antigène de Goodpasture (GPBP-1, *Goodpasture antigen binding protein-1*) pour l'utilisation dans le traitement d'un état prédiabétique.

2. Inhibiteur de GPBP-1 pour l'utilisation conforme à la revendication 1, le sujet présentant une valeur de GPBP-1 circulante plus élevée que celle d'un témoin, lequel témoin est constitué par un échantillon témoin prélevé sur un sujet normal, ou par des valeurs normales de GPBP-1 déterminées au préalable chez une population témoin.

3. Inhibiteur de GPBP-1 pour l'utilisation conforme à l'une quelconque des revendications 1 et 2, l'inhibiteur de GPBP-1 comprenant un anticorps monoclonal anti-GPBP-1, tel qu'un anticorps monoclonal humanisé.

4. Inhibiteur de GPBP-1 pour l'utilisation conforme à l'une quelconque des revendications 1 à 3, l'inhibiteur de GPBP-1 comprenant un composé de formule : 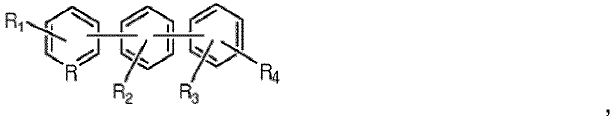 dans laquelle :
- R représente un atome d'azote ou un chaînon symbolisé par CR₅,
- R₅ représente une entité choisie dans l'ensemble constitué par les atomes d'hydrogène et d'halogène, et les groupes cyano, nitro, hydroxyle, alkyle en C₁-C₆, alcényle en C₂-C₆, alcynyle en C₂-C₆, halogéno-alkyle en C₁-C₆, alcoxy en C₁-C₆, halogéno-alcoxy en C₁-C₆, amino, alkyl-amino en C₁-C₆, di(alkyle en C₁-C₆)-amino, hydroxy-alkyle en C₁-C₆, (alcoxy en C₁-C₆)-(alkyle en C₁-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)(alcoxy en C₁-C₆), -(CH₂)₁₋₅-C(O)NH₂, aryl-(alkyle en C₂-C₆) et hétéroaryl-(alkyle en C₁-C₆),
- R₁ représente un atome d'hydrogène ou d'halogène, ou un groupe hydroxyle, alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), amino-alkyle en C₁-C₆, sulfanyl-alkyle en C₁-C₆ ou (alkyle en C₁-C₆)-sulfanyl-(alkyle en C₁-C₆),
- R₂ représente un groupe alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), formyl-(alkyle en C₀-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)OH, -(CH₂)₁₋₅-C(O)(alcoxy en C₁-C₆), -(CH₂)₁₋₅-C(O)NH₂, aryl-(alkyle en C₁-C₆) ou hétéroaryl-(alkyle en C₁-C₆),
- R₃ représente un groupe alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), formyl-(alkyle en C₁-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)OH, -(CH₂)₁₋₅-C(O)(alcoxy en C₁-C₆), -(CH₂)₁₋₅-C(O)NH₂, -(CH₂)₁₋₅-C(O)NH(alkyle en C₁-C₆), -(CH₂)₁₋₅-C(O)N(alkyle en C₁-C₆)₂, -CH=CH-C(O)OH, -CH=CH-C(O)(alcoxy en C₁-C₆), aryl-(alkyle en C₁-C₆) ou hétéroaryl-(alkyle en C₁-C₆),
- et R₄ représente un atome d'halogène ou un groupe hydroxyle, alkyle en C₁-C₆, alcoxy en C₁-C₆, halogéno-alcoxy en C₁-C₆, benzyloxy, -(CH₂)₁₋₅-C(O)OH, -(CH₂)₁₋₅-C(O)(alcoxy en C₁-C₆), -(CH₂)₁₋₅-C(O)NH₂, -(CH₂)₁₋₅-C(O)NH(alkyle en C₁-C₆), -(CH₂)₁₋₅-C(O)N(alkyle en C₁-C₆)₂, -CH=CH-C(O)OH, -CH=CH-C(O)(alcoxy en C₁-C₆), -O(CH₂)₁₋₅-C(O)OH, -O(CH₂)₁₋₅-C(O)(alcoxy en C₁-C₆), aryl-(alkyle en C₁-C₆) ou hétéroaryl-(alkyle en C₁-C₆),
ou un sel pharmacologiquement admissible d'un tel composé.

5. Inhibiteur de GPBP-1 pour l'utilisation conforme à l'une quelconque des revendications 1 à 4, l'inhibiteur de GPBP-1 comprenant un composé qui répond à la formule : 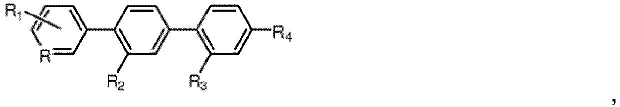 dans laquelle :
- R représente un atome d'azote ou un chaînon symbolisé par CR₅,
- R₅ représente une entité choisie dans l'ensemble constitué par les atomes d'hydrogène et d'halogène, et les groupes cyano, nitro, hydroxyle, alkyle en C₁-C₆, alcényle en C₂-C₆, alcynyle en C₂-C₆, halogéno-alkyle en C₁-C₆, alcoxy en C₁-C₆, halogéno-alcoxy en C₁-C₆, amino, alkyl-amino en C₁-C₆, di(alkyle en C₁-C₆)-amino, hydroxy-alkyle en C₁-C₆, (alcoxy en C₁-C₆)-(alkyle en C₁-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)(alcoxy en C₁-C₆) et -(CH₂)₁₋₅-C(O)NH₂,
- R₁ représente un atome d'hydrogène ou d'halogène, ou un groupe hydroxyle, alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), amino-alkyle en C₁-C₆, sulfanyl-alkyle en C₁-C₆ ou (alkyle en C₁-C₆)-sulfanyl-(alkyle en C₁-C₆),
- R₂ représente un groupe alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), formyl-(alkyle en C₀-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)OH, -(CH₂)₁₋₅-C(O)(alcoxy en C₁-C₆) ou -(CH₂)₁₋₅-C(O)NH₂,
- R₃ représente un groupe alkyle en C₁-C₆, halogéno-alkyle en C₁-C₆, alcoxy en C₁-C₆, halogéno-alcoxy en C₁-C₆, hydroxy-alkyle en C₁-C₆, (alcoxy en C₁-C₆)-(alkyle en C₁-C₆), formyl-(alkyle en C₁-C₆), amino-alkyle en C₁-C₆, sulfanyl-alkyle en C₁-C₆, (alkyle en C₁-C₆)-sulfanyl-(alkyle en C₁-C₆), -(CH₂)₁₋₅-C(O)OH, -(CH₂)₁₋₅-C(O)(alcoxy en C₁-C₆), -(CH₂)₁₋₅-C(O)NH₂, -(CH₂)₁₋₅-C(O)NH(alkyle en C₁-C₆), -(CH₂)₁₋₅-C(O)N(alkyle en C₁-C₆)₂, -CH=CH-C(O)OH ou -CH=CH-C(O)(alcoxy en C₁-C₆),
- et R₄ représente un atome d'halogène ou un groupe hydroxyle, alkyle en C₁-C₆, alcoxy en C₁-C₆, halogéno-alcoxy en C₁-C₆, benzyloxy, -(CH₂)₁₋₅-C(O)OH, -(CH₂)₁₋₅-C(O)(alcoxy en C₁-C₆), -(CH₂)₁₋₅-C(O)NH₂, -(CH₂)₁₋₅-C(O)NH(alkyle en C₁-C₆), -(CH₂)₁₋₅-C(O)N(alkyle en C₁-C₆)₂, -CH=CH-C(O)OH, -CH=CH-C(O)(alcoxy en C₁-C₆), -O(CH₂)₁₋₅-C(O)OH ou -O(CH₂)₁₋₅-C(O)(alcoxy en C₁-C₆),
ou un sel pharmacologiquement admissible d'un tel composé.

6. Inhibiteur de GPBP-1 pour l'utilisation conforme à l'une quelconque des revendications 1 à 5, l'inhibiteur de GPBP-1 comprenant de l'acide 3-[4"-méthoxy-3,2'-diméthyl-(1,1'; 4',1")-terphényl-2"-yl]-propionique, de l'acide 3-[2'-méthyl-4"-méthoxy-3-(trifluorométhyl)-(1,1' ; 4',1")-terphényl-2"-yl]-propionique, des combinaisons de tels composés, ou un sel pharmacologiquement admissible de tels composés.

7. Inhibiteur de GPBP-1 pour l'utilisation conforme à l'une quelconque des revendications 1 à 6, l'inhibiteur de GPBP-1 comprenant de l'acide 3-[4"-méthoxy-3,2'-diméthyl-(1,1' ; 4',1")-terphényl-2"-yl]-propionique ou un sel pharmacologiquement admissible d'un tel composé.

8. Inhibiteur de GPBP-1 pour l'utilisation conforme à l'une quelconque des revendications 1 à 7, l'inhibiteur de GPBP-1 comprenant un ou plusieurs inhibiteur(s) choisi(s) dans l'ensemble constitué par les suivants :
(a) un anticorps ou aptamère sélectif pour la protéine GPBP-1,
(c) un acide nucléique inhibiteur sélectif de l'ARNm de GPBP-1,
(d) un peptide comportant une séquence d'acides aminés de formule générale :
X1-SHClX2-X3 (SEQ ID NO : 1),
dans laquelle
- X1 représente 0 à 10 acides aminés appartenant à la séquence ATTAGILATL (SEQ ID NO : 2),
- X2 représente l'acide aminé E ou Q,
- et X3 représente 0 à 10 acides aminés appartenant à la séquence LMVKREDSWQ (SEQ ID NO : 3),
(e) un peptide comportant une séquence d'acides aminés choisie dans l'ensemble constitué par les séquences SHCIE (SEQ ID NO : 4), SHCIQ (SEQ ID NO: 5), ILATLSHCIELMVKR (SEQ ID NO: 6), ILATLSHCIQLMVKR (SEQ ID NO : 7) et LATLSHCIELMVKR (SEQ ID NO : 8),
(f) et I-20 :

9. Procédé pour diagnostiquer un état prédiabétique, comportant le fait de
(a) déterminer la quantité de GPBP-1 dans un échantillon prélevé sur un sujet risquant de présenter un état prédiabétique,
(b) et comparer la quantité de GPBP-1 dans cet échantillon avec la quantité de GPBP-1 dans un échantillon témoin, l'échantillon témoin étant un échantillon prélevé sur un sujet normal,
une quantité de GPBP-1 plus élevée dans l'échantillon que dans l'échantillon témoin indiquant l'existence d'un état prédiabétique chez le sujet.

10. Procédé pour diagnostiquer une disposition à développer un diabète de type 2, comportant le fait de
(a) déterminer la quantité de GPBP-1 dans un échantillon prélevé sur un sujet risquant de contracter un diabète de type 2,
(b) et comparer la quantité de GPBP-1 dans cet échantillon avec la quantité de GPBP-1 dans un échantillon témoin, l'échantillon témoin étant un échantillon prélevé sur un sujet normal,
une quantité de GPBP-1 plus élevée dans l'échantillon que dans l'échantillon témoin indiquant une disposition à développer un diabète de type 2 chez le sujet.

11. Procédé selon la revendication 9 ou la revendication 10, dans lequel l'établissement d'un diagnostic comporte le fait de
(a) mettre un échantillon de plasma prélevé sur le sujet, en contact avec une molécule de liaison à la GPBP-1, qui se lie à la protéine GPBP-1 dans des conditions favorisant une liaison sélective de la molécule de liaison à la GPBP-1 avec la protéine GPBP-1,
(b) éliminer le plasma non lié et/ou les molécules de liaison à la GPBP-1 non liées,
(c) et détecter la formation d'un complexe entre la protéine GPBP-1 et la molécule de liaison à la GPBP-1 au sein de l'échantillon de plasma,
la formation d'un complexe fournissant une valeur de mesure de la protéine GPBP-1 dans l'échantillon.

12. Procédé selon l'une quelconque des revendications 9 à 11, dans lequel la molécule de liaison à la GPBP-1 comprend un anticorps, tel qu'un anticorps monoclonal.


### SEQUENCE LISTING

<110> FibroStatin, S.L.
<120> Goodpasture Antigen Binding Protein Detection and Inhibition and its Use in Diabetes
<130> 150-338 PCT
<150> 61/883792
   <151> 2013-09-27
<160> 25
<170> PatentIn version 3.5
<210> 1
   <211> 25
   <212> PRT
   <213> Artificial Sequence
<220>
   <223> Synthetic
<220>
   <221> MISC_FEATURE
   <222> (1)..(10)
   <223> Optionally absent
<220>
   <221> MISC_FEATURE
   <222> (15)..(15)
   <223> Xaa is E or Q
<220>
   <221> MISC_FEATURE
   <222> (16)..(25)
   <223> Optionally absent
<400> 1
   Ala Thr Thr Ala Gly Ile Leu Ala Thr Leu Ser His Cys Ile Xaa Leu
   1               5                   10                  15
   Met Val Lys Arg Glu Asp Ser Trp Gln
               20                  25
<210> 2
   <211> 10
   <212> PRT
   <213> Artificial Sequence
<220>
   <223> Synthetic
<400> 2
   Ala Thr Thr Ala Gly Ile Leu Ala Thr Leu
   1               5                   10
<210> 3
   <211> 10
   <212> PRT
   <213> Artificial Sequence
<220>
   <223> Synthetic
<400> 3
   Leu Met Val Lys Arg Glu Asp Ser Trp Gln
   1               5                   10
<210> 4
   <211> 5
   <212> PRT
   <213> Artificial Sequence
<220>
   <223> Synthetic
<400> 4
   Ser His Cys Ile Glu
   1               5
<210> 5
   <211> 5
   <212> PRT
   <213> Artificial Sequence
<220>
   <223> Synthetic
<400> 5
   Ser His Cys Ile Gln
   1               5
<210> 6
   <211> 15
   <212> PRT
   <213> Artificial Sequence
<220>
   <223> Synthetic
<400> 6
   Ile Leu Ala Thr Leu Ser His Cys Ile Glu Leu Met Val Lys Arg
   1               5                   10                  15
<210> 7
   <211> 15
   <212> PRT
   <213> Artificial Sequence
<220>
   <223> Synthetic
<400> 7
   Ile Leu Ala Thr Leu Ser His Cys Ile Gln Leu Met Val Lys Arg
   1               5                   10                  15
<210> 8
   <211> 14
   <212> PRT
   <213> Artificial Sequence
<220>
   <223> Synthetic
<400> 8
   Leu Ala Thr Leu Ser His Cys Ile Glu Leu Met Val Lys Arg
   1               5                   10
<210> 9
   <211> 265
   <212> PRT
   <213> Artificial Sequence
<220>
   <223> Synthetic
<400> 9
   Arg Asp Glu Val Ile Gly Ile Leu Lys Ala Glu Lys Met Asp Leu Ala
   1               5                   10                  15
   Leu Leu Glu Ala Gln Tyr Gly Phe Val Thr Pro Lys Lys Val Leu Glu
               20                  25                  30
   Ala Leu Gln Arg Asp Ala Phe Gln Ala Lys Ser Thr Pro Trp Gln Glu
           35                  40                  45
   Asp Ile Tyr Glu Lys Pro Met Asn Glu Leu Asp Lys Val Val Glu Lys
       50                  55                  60
   His Lys Glu Ser Tyr Arg Arg Ile Leu Gly Gln Leu Leu Val Ala Glu
   65                  70                  75                  80
   Lys Ser Arg Arg Gln Thr Ile Leu Glu Leu Glu Glu Glu Lys Arg Lys
                   85                  90                  95
   His Lys Glu Tyr Met Glu Lys Ser Asp Glu Phe Ile Cys Leu Leu Glu
               100                 105                 110
   Gln Glu Cys Glu Arg Leu Lys Lys Leu Ile Asp Gln Glu Ile Lys Ser
           115                 120                 125
   Gln Glu Glu Lys Glu Gln Glu Lys Glu Lys Arg Val Thr Thr Leu Lys
       130                 135                 140
   Glu Glu Leu Thr Lys Leu Lys Ser Phe Ala Leu Met Val Val Asp Glu
   145                 150                 155                 160
   Gln Gln Arg Leu Thr Ala Gln Leu Thr Leu Gln Arg Gln Lys Ile Gln
                   165                 170                 175
   Glu Leu Thr Thr Asn Ala Lys Glu Thr His Thr Lys Leu Ala Leu Ala
               180                 185                 190
   Glu Ala Arg Val Gln Glu Glu Glu Gln Lys Ala Thr Arg Leu Glu Lys
           195                 200                 205
   Glu Leu Gln Thr Gln Thr Thr Lys Phe His Gln Asp Gln Asp Thr Ile
       210                 215                 220
   Met Ala Lys Leu Thr Asn Glu Asp Ser Gln Asn Arg Gln Leu Gln Gln
   225                 230                 235                 240
   Lys Leu Ala Ala Leu Ser Arg Gln Ile Asp Glu Leu Glu Glu Thr Asn
                   245                 250                 255
   Arg Ser Leu Arg Lys Ala Glu Glu Glu
               260                 265
<210> 10
   <211> 624
   <212> PRT
   <213> Homo sapiens
<400> 10
   Met Ser Asp Asn Gln Ser Trp Asn Ser Ser Gly Ser Glu Glu Asp Pro
   1               5                   10                  15
   Glu Thr Glu Ser Gly Pro Pro Val Glu Arg Cys Gly Val Leu Ser Lys
               20                  25                  30
   Trp Thr Asn Tyr Ile His Gly Trp Gln Asp Arg Trp Val Val Leu Lys
           35                  40                  45
   Asn Asn Ala Leu Ser Tyr Tyr Lys Ser Glu Asp Glu Thr Glu Tyr Gly
       50                  55                  60
   Cys Arg Gly Ser Ile Cys Leu Ser Lys Ala Val Ile Thr Pro His Asp
   65                  70                  75                  80
   Phe Asp Glu Cys Arg Phe Asp Ile Ser Val Asn Asp Ser Val Trp Tyr
                   85                  90                  95
   Leu Arg Ala Gln Asp Pro Asp His Arg Gln Gln Trp Ile Asp Ala Ile
               100                 105                 110
   Glu Gln His Lys Thr Glu Ser Gly Tyr Gly Ser Glu Ser Ser Leu Arg
           115                 120                 125
   Arg His Gly Ser Met Val Ser Leu Val Ser Gly Ala Ser Gly Tyr Ser
       130                 135                 140
   Ala Thr Ser Thr Ser Ser Phe Lys Lys Gly His Ser Leu Arg Glu Lys
   145                 150                 155                 160
   Leu Ala Glu Met Glu Thr Phe Arg Asp Ile Leu Cys Arg Gln Val Asp
                   165                 170                 175
   Thr Leu Gln Lys Tyr Phe Asp Ala Cys Ala Asp Ala Val Ser Lys Asp
               180                 185                 190
   Glu Leu Gln Arg Asp Lys Val Val Glu Asp Asp Glu Asp Asp Phe Pro
           195                 200                 205
   Thr Thr Arg Ser Asp Gly Asp Phe Leu His Ser Thr Asn Gly Asn Lys
       210                 215                 220
   Glu Lys Leu Phe Pro His Val Thr Pro Lys Gly Ile Asn Gly Ile Asp
   225                 230                 235                 240
   Phe Lys Gly Glu Ala Ile Thr Phe Lys Ala Thr Thr Ala Gly Ile Leu
                   245                 250                 255
   Ala Thr Leu Ser His Cys Ile Glu Leu Met Val Lys Arg Glu Asp Ser
               260                 265                 270
   Trp Gln Lys Arg Leu Asp Lys Glu Thr Glu Lys Lys Arg Arg Thr Glu
           275                 280                 285
   Glu Ala Tyr Lys Asn Ala Met Thr Glu Leu Lys Lys Lys Ser His Phe
       290                 295                 300
   Gly Gly Pro Asp Tyr Glu Glu Gly Pro Asn Ser Leu Ile Asn Glu Glu
   305                 310                 315                 320
   Glu Phe Phe Asp Ala Val Glu Ala Ala Leu Asp Arg Gln Asp Lys Ile
   325 330 335
   Glu Glu Gln Ser Gln Ser Glu Lys Val Arg Leu His Trp Pro Thr Ser
   340 345 350
   Leu Pro Ser Gly Asp Ala Phe Ser Ser Val Gly Thr His Arg Phe Val
   355 360 365
   Gln Lys Pro Tyr Ser Arg Ser Ser Ser Met Ser Ser Ile Asp Leu Val
   370 375 380
   Ser Ala Ser Asp Asp Val His Arg Phe Ser Ser Gln Val Glu Glu Met
   385 390 395 400
   Val Gln Asn His Met Thr Tyr Ser Leu Gln Asp Val Gly Gly Asp Ala
   405 410 415
   Asn Trp Gln Leu Val Val Glu Glu Gly Glu Met Lys Val Tyr Arg Arg
   420 425 430
   Glu Val Glu Glu Asn Gly Ile Val Leu Asp Pro Leu Lys Ala Thr His
   435 440 445
   Ala Val Lys Gly Val Thr Gly His Glu Val Cys Asn Tyr Phe Trp Asn
   450 455 460
   Val Asp Val Arg Asn Asp Trp Glu Thr Thr Ile Glu Asn Phe His Val
   465 470 475 480
   Val Glu Thr Leu Ala Asp Asn Ala Ile Ile Ile Tyr Gln Thr His Lys
   485 490 495
   Arg Val Trp Pro Ala Ser Gln Arg Asp Val Leu Tyr Leu Ser Val Ile
   500 505 510
   Arg Lys Ile Pro Ala Leu Thr Glu Asn Asp Pro Glu Thr Trp Ile Val
   515 520 525
   Cys Asn Phe Ser Val Asp His Asp Ser Ala Pro Leu Asn Asn Arg Cys
   530 535 540
   Val Arg Ala Lys Ile Asn Val Ala Met Ile Cys Gln Thr Leu Val Ser
   545 550 555 560
   Pro Pro Glu Gly Asn Gln Glu Ile Ser Arg Asp Asn Ile Leu Cys Lys
   565 570 575
   Ile Thr Tyr Val Ala Asn Val Asn Pro Gly Gly Trp Ala Pro Ala Ser
   580 585 590
   Val Leu Arg Ala Val Ala Lys Arg Glu Tyr Pro Lys Phe Leu Lys Arg
   595 600 605
   Phe Thr Ser Tyr Val Gln Glu Lys Thr Ala Gly Lys Pro Ile Leu Phe
   610 615 620
<210> 11
   <211> 598
   <212> PRT
   <213> Homo sapiens
<400> 11
   Met Ser Asp Asn Gln Ser Trp Asn Ser Ser Gly Ser Glu Glu Asp Pro
   1 5 10 15
   Glu Thr Glu Ser Gly Pro Pro Val Glu Arg Cys Gly Val Leu Ser Lys
   20 25 30
   Trp Thr Asn Tyr Ile His Gly Trp Gln Asp Arg Trp Val Val Leu Lys
   35 40 45
   Asn Asn Ala Leu Ser Tyr Tyr Lys Ser Glu Asp Glu Thr Glu Tyr Gly
   50 55 60
   Cys Arg Gly Ser Ile Cys Leu Ser Lys Ala Val Ile Thr Pro His Asp
   65 70 75 80
   Phe Asp Glu Cys Arg Phe Asp Ile Ser Val Asn Asp Ser Val Trp Tyr
   85 90 95
   Leu Arg Ala Gln Asp Pro Asp His Arg Gln Gln Trp Ile Asp Ala Ile
   100 105 110
   Glu Gln His Lys Thr Glu Ser Gly Tyr Gly Ser Glu Ser Ser Leu Arg
   115 120 125
   Arg His Gly Ser Met Val Ser Leu Val Ser Gly Ala Ser Gly Tyr Ser
   130 135 140
   Ala Thr Ser Thr Ser Ser Phe Lys Lys Gly His Ser Leu Arg Glu Lys
   145 150 155 160
   Leu Ala Glu Met Glu Thr Phe Arg Asp Ile Leu Cys Arg Gln Val Asp
   165 170 175
   Thr Leu Gln Lys Tyr Phe Asp Ala Cys Ala Asp Ala Val Ser Lys Asp
               180                 185                 190
   Glu Leu Gln Arg Asp Lys Val Val Glu Asp Asp Glu Asp Asp Phe Pro
           195                 200                 205
   Thr Thr Arg Ser Asp Gly Asp Phe Leu His Ser Thr Asn Gly Asn Lys
       210                 215                 220
   Glu Lys Leu Phe Pro His Val Thr Pro Lys Gly Ile Asn Gly Ile Asp
   225                 230                 235                 240
   Phe Lys Gly Glu Ala Ile Thr Phe Lys Ala Thr Thr Ala Gly Ile Leu
                   245                 250                 255
   Ala Thr Leu Ser His Cys Ile Glu Leu Met Val Lys Arg Glu Asp Ser
               260                 265                 270
   Trp Gln Lys Arg Leu Asp Lys Glu Thr Glu Lys Lys Arg Arg Thr Glu
           275                 280                 285
   Glu Ala Tyr Lys Asn Ala Met Thr Glu Leu Lys Lys Lys Ser His Phe
       290                 295                 300
   Gly Gly Pro Asp Tyr Glu Glu Gly Pro Asn Ser Leu Ile Asn Glu Glu
   305                 310                 315                 320
   Glu Phe Phe Asp Ala Val Glu Ala Ala Leu Asp Arg Gln Asp Lys Ile
                   325                 330                 335
   Glu Glu Gln Ser Gln Ser Glu Lys Val Arg Leu His Trp Pro Thr Ser
               340                 345                 350
   Leu Pro Ser Gly Asp Ala Phe Ser Ser Val Gly Thr His Arg Phe Val
           355                 360                 365
   Gln Lys Val Glu Glu Met Val Gln Asn His Met Thr Tyr Ser Leu Gln
       370                 375                 380
   Asp Val Gly Gly Asp Ala Asn Trp Gln Leu Val Val Glu Glu Gly Glu
   385                 390                 395                 400
   Met Lys Val Tyr Arg Arg Glu Val Glu Glu Asn Gly Ile Val Leu Asp
                   405                 410                 415
   Pro Leu Lys Ala Thr His Ala Val Lys Gly Val Thr Gly His Glu Val
               420                 425                 430
   Cys Asn Tyr Phe Trp Asn Val Asp Val Arg Asn Asp Trp Glu Thr Thr
           435                 440                 445
   Ile Glu Asn Phe His Val Val Glu Thr Leu Ala Asp Asn Ala Ile Ile
       450                 455                 460
   Ile Tyr Gln Thr His Lys Arg Val Trp Pro Ala Ser Gln Arg Asp Val
   465                 470                 475                 480
   Leu Tyr Leu Ser Val Ile Arg Lys Ile Pro Ala Leu Thr Glu Asn Asp
                   485                 490                 495
   Pro Glu Thr Trp Ile Val Cys Asn Phe Ser Val Asp His Asp Ser Ala
               500                 505                 510
   Pro Leu Asn Asn Arg Cys Val Arg Ala Lys Ile Asn Val Ala Met Ile
           515                 520                 525
   Cys Gln Thr Leu Val Ser Pro Pro Glu Gly Asn Gln Glu Ile Ser Arg
       530                 535                 540
   Asp Asn Ile Leu Cys Lys Ile Thr Tyr Val Ala Asn Val Asn Pro Gly
   545                 550                 555                 560
   Gly Trp Ala Pro Ala Ser Val Leu Arg Ala Val Ala Lys Arg Glu Tyr
                   565                 570                 575
   Pro Lys Phe Leu Lys Arg Phe Thr Ser Tyr Val Gln Glu Lys Thr Ala
               580                 585                 590
   Gly Lys Pro Ile Leu Phe
           595
<210> 12
   <211> 707
   <212> PRT
   <213> Homo sapiens
<400> 12
   Thr Ala Ala Ala Ala Asp Gly Trp Lys Gly Arg Leu Pro Ser Pro Leu
   1               5                   10                  15
   Val Leu Leu Pro Arg Ser Ala Arg Cys Gln Ala Arg Arg Arg Arg Gly
               20                  25                  30
   Gly Arg Thr Ser Ser Leu Leu Leu Leu Pro Pro Thr Pro Glu Arg Ala
           35                  40                  45
   Leu Phe Ala Ser Pro Ser Pro Asp Pro Ser Pro Arg Gly Leu Gly Ala
       50                  55                  60
   Ser Ser Gly Ala Ala Glu Gly Ala Gly Ala Gly Leu Leu Leu Gly Cys
   65                  70                  75                  80
   Arg Ala Ser Met Ser Asp Asn Gln Ser Trp Asn Ser Ser Gly Ser Glu
                   85                  90                  95
   Glu Asp Pro Glu Thr Glu Ser Gly Pro Pro Val Glu Arg Cys Gly Val
               100                 105                 110
   Leu Ser Lys Trp Thr Asn Tyr Ile His Gly Trp Gln Asp Arg Trp Val
           115                 120                 125
   Val Leu Lys Asn Asn Ala Leu Ser Tyr Tyr Lys Ser Glu Asp Glu Thr
       130                 135                 140
   Glu Tyr Gly Cys Arg Gly Ser Ile Cys Leu Ser Lys Ala Val Ile Thr
   145                 150                 155                 160
   Pro His Asp Phe Asp Glu Cys Arg Phe Asp Ile Ser Val Asn Asp Ser
                   165                 170                 175
   Val Trp Tyr Leu Arg Ala Gln Asp Pro Asp His Arg Gln Gln Trp Ile
               180                 185                 190
   Asp Ala Ile Glu Gln His Lys Thr Glu Ser Gly Tyr Gly Ser Glu Ser
           195                 200                 205
   Ser Leu Arg Arg His Gly Ser Met Val Ser Leu Val Ser Gly Ala Ser
       210                 215                 220
   Gly Tyr Ser Ala Thr Ser Thr Ser Ser Phe Lys Lys Gly His Ser Leu
   225                 230                 235                 240
   Arg Glu Lys Leu Ala Glu Met Glu Thr Phe Arg Asp Ile Leu Cys Arg
                   245                 250                 255
   Gln Val Asp Thr Leu Gln Lys Tyr Phe Asp Ala Cys Ala Asp Ala Val
               260                 265                 270
   Ser Lys Asp Glu Leu Gln Arg Asp Lys Val Val Glu Asp Asp Glu Asp
           275                 280                 285
   Asp Phe Pro Thr Thr Arg Ser Asp Gly Asp Phe Leu His Ser Thr Asn
       290                 295                 300
   Gly Asn Lys Glu Lys Leu Phe Pro His Val Thr Pro Lys Gly Ile Asn
   305                 310                 315                 320
   Gly Ile Asp Phe Lys Gly Glu Ala Ile Thr Phe Lys Ala Thr Thr Ala
                   325                 330                 335
   Gly Ile Leu Ala Thr Leu Ser His Cys Ile Glu Leu Met Val Lys Arg
               340                 345                 350
   Glu Asp Ser Trp Gln Lys Arg Leu Asp Lys Glu Thr Glu Lys Lys Arg
           355                 360                 365
   Arg Thr Glu Glu Ala Tyr Lys Asn Ala Met Thr Glu Leu Lys Lys Lys
       370                 375                 380
   Ser His Phe Gly Gly Pro Asp Tyr Glu Glu Gly Pro Asn Ser Leu Ile
   385                 390                 395                 400
   Asn Glu Glu Glu Phe Phe Asp Ala Val Glu Ala Ala Leu Asp Arg Gln
                   405                 410                 415
   Asp Lys Ile Glu Glu Gln Ser Gln Ser Glu Lys Val Arg Leu His Trp
               420                 425                 430
   Pro Thr Ser Leu Pro Ser Gly Asp Ala Phe Ser Ser Val Gly Thr His
           435                 440                 445
   Arg Phe Val Gln Lys Pro Tyr Ser Arg Ser Ser Ser Met Ser Ser Ile
       450                 455                 460
   Asp Leu Val Ser Ala Ser Asp Asp Val His Arg Phe Ser Ser Gln Val
   465                 470                 475                 480
   Glu Glu Met Val Gln Asn His Met Thr Tyr Ser Leu Gln Asp Val Gly
                   485                 490                 495
   Gly Asp Ala Asn Trp Gln Leu Val Val Glu Glu Gly Glu Met Lys Val
               500                 505                 510
   Tyr Arg Arg Glu Val Glu Glu Asn Gly Ile Val Leu Asp Pro Leu Lys
           515                 520                 525
   Ala Thr His Ala Val Lys Gly Val Thr Gly His Glu Val Cys Asn Tyr
       530                 535                 540
   Phe Trp Asn Val Asp Val Arg Asn Asp Trp Glu Thr Thr Ile Glu Asn
   545 550 555 560
   Phe His Val Val Glu Thr Leu Ala Asp Asn Ala Ile Ile Ile Tyr Gln
   565 570 575
   Thr His Lys Arg Val Trp Pro Ala Ser Gln Arg Asp Val Leu Tyr Leu
   580 585 590
   Ser Val Ile Arg Lys Ile Pro Ala Leu Thr Glu Asn Asp Pro Glu Thr
   595 600 605
   Trp Ile Val Cys Asn Phe Ser Val Asp His Asp Ser Ala Pro Leu Asn
   610 615 620
   Asn Arg Cys Val Arg Ala Lys Ile Asn Val Ala Met Ile Cys Gln Thr
   625 630 635 640
   Leu Val Ser Pro Pro Glu Gly Asn Gln Glu Ile Ser Arg Asp Asn Ile
   645 650 655
   Leu Cys Lys Ile Thr Tyr Val Ala Asn Val Asn Pro Gly Gly Trp Ala
   660 665 670
   Pro Ala Ser Val Leu Arg Ala Val Ala Lys Arg Glu Tyr Pro Lys Phe
   675 680 685
   Leu Lys Arg Phe Thr Ser Tyr Val Gln Glu Lys Thr Ala Gly Lys Pro
   690 695 700
   Ile Leu Phe
   705
<210> 13
<400> 13
   000
<210> 14
   <211> 764
   <212> PRT
   <213> Homo sapiens
<400> 14
   Met Arg Ser Arg Gly Ser Asp Thr Glu Gly Ser Ala Gln Lys Lys Phe
   1 5 10 15
   Pro Arg His Thr Lys Gly His Ser Phe Gln Gly Pro Lys Asn Met Lys
   20 25 30
   His Arg Gln Gln Asp Lys Asp Ser Pro Ser Glu Ser Asp Val Ile Leu
           35                  40                  45
   Pro Cys Pro Lys Ala Glu Lys Pro His Ser Gly Asn Gly His Gln Ala
       50                  55                  60
   Glu Asp Leu Ser Arg Asp Asp Leu Leu Phe Leu Leu Ser Ile Leu Glu
   65                  70                  75                  80
   Gly Glu Leu Gln Ala Arg Asp Glu Val Ile Gly Ile Leu Lys Ala Glu
                   85                  90                  95
   Lys Met Asp Leu Ala Leu Leu Glu Ala Gln Tyr Gly Phe Val Thr Pro
               100                 105                 110
   Lys Lys Val Leu Glu Ala Leu Gln Arg Asp Ala Phe Gln Ala Lys Ser
           115                 120                 125
   Thr Pro Trp Gln Glu Asp Ile Tyr Glu Lys Pro Met Asn Glu Leu Asp
       130                 135                 140
   Lys Val Val Glu Lys His Lys Glu Ser Tyr Arg Arg Ile Leu Gly Gln
   145                 150                 155                 160
   Leu Leu Val Ala Glu Lys Ser Arg Arg Gln Thr Ile Leu Glu Leu Glu
                   165                 170                 175
   Glu Glu Lys Arg Lys His Lys Glu Tyr Met Glu Lys Ser Asp Glu Phe
               180                 185                 190
   Ile Cys Leu Leu Glu Gln Glu Cys Glu Arg Leu Lys Lys Leu Ile Asp
           195                 200                 205
   Gln Glu Ile Lys Ser Gln Glu Glu Lys Glu Gln Glu Lys Glu Lys Arg
       210                 215                 220
   Val Thr Thr Leu Lys Glu Glu Leu Thr Lys Leu Lys Ser Phe Ala Leu
   225                 230                 235                 240
   Met Val Val Asp Glu Gln Gln Arg Leu Thr Ala Gln Leu Thr Leu Gln
                   245                 250                 255
   Arg Gln Lys Ile Gln Glu Leu Thr Thr Asn Ala Lys Glu Thr His Thr
               260                 265                 270
   Lys Leu Ala Leu Ala Glu Ala Arg Val Gln Glu Glu Glu Gln Lys Ala
           275                 280                 285
   Thr Arg Leu Glu Lys Glu Leu Gln Thr Gln Thr Thr Lys Phe His Gln
       290                 295                 300
   Asp Gln Asp Thr Ile Met Ala Lys Leu Thr Asn Glu Asp Ser Gln Asn
   305                 310                 315                 320
   Arg Gln Leu Gln Gln Lys Leu Ala Ala Leu Ser Arg Gln Ile Asp Glu
                   325                 330                 335
   Leu Glu Glu Thr Asn Arg Ser Leu Arg Lys Ala Glu Glu Glu Leu Gln
               340                 345                 350
   Asp Ile Lys Glu Lys Ile Ser Lys Gly Glu Tyr Gly Asn Ala Gly Ile
           355                 360                 365
   Met Ala Glu Val Glu Glu Leu Arg Lys Arg Val Leu Asp Met Glu Gly
       370                 375                 380
   Lys Asp Glu Glu Leu Ile Lys Met Glu Glu Gln Cys Arg Asp Leu Asn
   385                 390                 395                 400
   Lys Arg Leu Glu Arg Glu Thr Leu Gln Ser Lys Asp Phe Lys Leu Glu
                   405                 410                 415
   Val Glu Lys Leu Ser Lys Arg Ile Met Ala Leu Glu Lys Leu Glu Asp
               420                 425                 430
   Ala Phe Asn Lys Ser Lys Gln Glu Cys Tyr Ser Leu Lys Cys Asn Leu
           435                 440                 445
   Glu Lys Glu Arg Met Thr Thr Lys Gln Leu Ser Gln Glu Leu Glu Ser
       450                 455                 460
   Leu Lys Val Arg Ile Lys Glu Leu Glu Ala Ile Glu Ser Arg Leu Glu
   465                 470                 475                 480
   Lys Thr Glu Phe Thr Leu Lys Glu Asp Leu Thr Lys Leu Lys Thr Leu
                   485                 490                 495
   Thr Val Met Phe Val Asp Glu Arg Lys Thr Met Ser Glu Lys Leu Lys
               500                 505                 510
   Lys Thr Glu Asp Lys Leu Gln Ala Ala Ser Ser Gln Leu Gln Val Glu
           515                 520                 525
   Gln Asn Lys Val Thr Thr Val Thr Glu Lys Leu Ile Glu Glu Thr Lys
       530                 535                 540
   Arg Ala Leu Lys Ser Lys Thr Asp Val Glu Glu Lys Met Tyr Ser Val
   545                 550                 555                 560
   Thr Lys Glu Arg Asp Asp Leu Lys Asn Lys Leu Lys Ala Glu Glu Glu
                   565                 570                 575
   Lys Gly Asn Asp Leu Leu Ser Arg Val Asn Met Leu Lys Asn Arg Leu
               580                 585                 590
   Gln Ser Leu Glu Ala Ile Glu Lys Asp Phe Leu Lys Asn Lys Leu Asn
           595                 600                 605
   Gln Asp Ser Gly Lys Ser Thr Thr Ala Leu His Gln Glu Asn Asn Lys
       610                 615                 620
   Ile Lys Glu Leu Ser Gln Glu Val Glu Arg Leu Lys Leu Lys Leu Lys
   625                 630                 635                 640
   Asp Met Lys Ala Ile Glu Asp Asp Leu Met Lys Thr Glu Asp Glu Tyr
                   645                 650                 655
   Glu Thr Leu Glu Arg Arg Tyr Ala Asn Glu Arg Asp Lys Ala Gln Phe
               660                 665                 670
   Leu Ser Lys Glu Leu Glu His Val Lys Met Glu Leu Ala Lys Tyr Lys
           675                 680                 685
   Leu Ala Glu Lys Thr Glu Thr Ser His Glu Gln Trp Leu Phe Lys Arg
       690                 695                 700
   Leu Gln Glu Glu Glu Ala Lys Ser Gly His Leu Ser Arg Glu Val Asp
   705                 710                 715                 720
   Ala Leu Lys Glu Lys Ile His Glu Tyr Met Ala Thr Glu Asp Leu Ile
                   725                 730                 735
   Cys His Leu Gln Gly Asp His Ser Val Leu Gln Lys Lys Thr Lys Ser
               740                 745                 750
   Thr Arg Lys Gln Glu Gln Arg Phe Arg Lys Arg Asp
           755                 760
<210> 15
   <211> 1135
   <212> PRT
   <213> Homo sapiens
<400> 15
   Met Arg Ser Arg Gly Ser Asp Thr Glu Gly Ser Ala Gln Lys Lys Phe
   1               5                   10                  15
   Pro Arg His Thr Lys Gly His Ser Phe Gln Gly Pro Lys Asn Met Lys
               20                  25                  30
   His Arg Gln Gln Asp Lys Asp Ser Pro Ser Glu Ser Asp Val Ile Leu
           35                  40                  45
   Pro Cys Pro Lys Ala Glu Lys Pro His Ser Gly Asn Gly His Gln Ala
       50                  55                  60
   Glu Asp Leu Ser Arg Asp Asp Leu Leu Phe Leu Leu Ser Ile Leu Glu
   65                  70                  75                  80
   Gly Glu Leu Gln Ala Arg Asp Glu Val Ile Gly Ile Leu Lys Ala Glu
                   85                  90                  95
   Lys Met Asp Leu Ala Leu Leu Glu Ala Gln Tyr Gly Phe Val Thr Pro
               100                 105                 110
   Lys Lys Val Leu Glu Ala Leu Gln Arg Asp Ala Phe Gln Ala Lys Ser
           115                 120                 125
   Thr Pro Trp Gln Glu Asp Ile Tyr Glu Lys Pro Met Asn Glu Leu Asp
       130                 135                 140
   Lys Val Val Glu Lys His Lys Glu Ser Tyr Arg Arg Ile Leu Gly Gln
   145                 150                 155                 160
   Leu Leu Val Ala Glu Lys Ser Arg Arg Gln Thr Ile Leu Glu Leu Glu
                   165                 170                 175
   Glu Glu Lys Arg Lys His Lys Glu Tyr Met Glu Lys Ser Asp Glu Phe
               180                 185                 190
   Ile Cys Leu Leu Glu Gln Glu Cys Glu Arg Leu Lys Lys Leu Ile Asp
           195                 200                 205
   Gln Glu Ile Lys Ser Gln Glu Glu Lys Glu Gln Glu Lys Glu Lys Arg
       210                 215                 220
   Val Thr Thr Leu Lys Glu Glu Leu Thr Lys Leu Lys Ser Phe Ala Leu
   225                 230                 235                 240
   Met Val Val Asp Glu Gln Gln Arg Leu Thr Ala Gln Leu Thr Leu Gln
   245 250 255
   Arg Gln Lys Ile Gln Glu Leu Thr Thr Asn Ala Lys Glu Thr His Thr
   260 265 270
   Lys Leu Ala Leu Ala Glu Ala Arg Val Gln Glu Glu Glu Gln Lys Ala
   275 280 285
   Thr Arg Leu Glu Lys Glu Leu Gln Thr Gln Thr Thr Lys Phe His Gln
   290 295 300
   Asp Gln Asp Thr Ile Met Ala Lys Leu Thr Asn Glu Asp Ser Gln Asn
   305 310 315 320
   Arg Gln Leu Gln Gln Lys Leu Ala Ala Leu Ser Arg Gln Ile Asp Glu
   325 330 335
   Leu Glu Glu Thr Asn Arg Ser Leu Arg Lys Ala Glu Glu Glu Leu Gln
   340 345 350
   Asp Ile Lys Glu Lys Ile Ser Lys Gly Glu Tyr Gly Asn Ala Gly Ile
   355 360 365
   Met Ala Glu Val Glu Glu Leu Arg Lys Arg Val Leu Asp Met Glu Gly
   370 375 380
   Lys Asp Glu Glu Leu Ile Lys Met Glu Glu Gln Cys Arg Asp Leu Asn
   385 390 395 400
   Lys Arg Leu Glu Arg Glu Thr Leu Gln Ser Lys Asp Phe Lys Leu Glu
   405 410 415
   Val Glu Lys Leu Ser Lys Arg Ile Met Ala Leu Glu Lys Leu Glu Asp
   420 425 430
   Ala Phe Asn Lys Ser Lys Gln Glu Cys Tyr Ser Leu Lys Cys Asn Leu
   435 440 445
   Glu Lys Glu Arg Met Thr Thr Lys Gln Leu Ser Gln Glu Leu Glu Ser
   450 455 460
   Leu Lys Val Arg Ile Lys Glu Leu Glu Ala Ile Glu Ser Arg Leu Glu
   465 470 475 480
   Lys Thr Glu Phe Thr Leu Lys Glu Asp Leu Thr Lys Leu Lys Thr Leu
   485 490 495
   Thr Val Met Phe Val Asp Glu Arg Lys Thr Met Ser Glu Lys Leu Lys
               500                 505                 510
   Lys Thr Glu Asp Lys Leu Gln Ala Ala Ser Ser Gln Leu Gln Val Glu
           515                 520                 525
   Gln Asn Lys Val Thr Thr Val Thr Glu Lys Leu Ile Glu Glu Thr Lys
       530                 535                 540
   Arg Ala Leu Lys Ser Lys Thr Asp Val Glu Glu Lys Met Tyr Ser Val
   545                 550                 555                 560
   Thr Lys Glu Arg Asp Asp Leu Lys Asn Lys Leu Lys Ala Glu Glu Glu
                   565                 570                 575
   Lys Gly Asn Asp Leu Leu Ser Arg Val Asn Met Leu Lys Asn Arg Leu
               580                 585                 590
   Gln Ser Leu Glu Ala Ile Glu Lys Asp Phe Leu Lys Asn Lys Leu Asn
           595                 600                 605
   Gln Asp Ser Gly Lys Ser Thr Thr Ala Leu His Gln Glu Asn Asn Lys
       610                 615                 620
   Ile Lys Glu Leu Ser Gln Glu Val Glu Arg Leu Lys Leu Lys Leu Lys
   625                 630                 635                 640
   Asp Met Lys Ala Ile Glu Asp Asp Leu Met Lys Thr Glu Asp Glu Tyr
                   645                 650                 655
   Glu Thr Leu Glu Arg Arg Tyr Ala Asn Glu Arg Asp Lys Ala Gln Phe
               660                 665                 670
   Leu Ser Lys Glu Leu Glu His Val Lys Met Glu Leu Ala Lys Tyr Lys
           675                 680                 685
   Leu Ala Glu Lys Thr Glu Thr Ser His Glu Gln Trp Leu Phe Lys Arg
       690                 695                 700
   Leu Gln Glu Glu Glu Ala Lys Ser Gly His Leu Ser Arg Glu Val Asp
   705                 710                 715                 720
   Ala Leu Lys Glu Lys Ile His Glu Tyr Met Ala Thr Glu Asp Leu Ile
                   725                 730                 735
   Cys His Leu Gln Gly Asp His Ser Val Leu Gln Lys Lys Leu Asn Gln
               740                 745                 750
   Gln Glu Asn Arg Asn Arg Asp Leu Gly Arg Glu Ile Glu Asn Leu Thr
           755                 760                 765
   Lys Glu Leu Glu Arg Tyr Arg His Phe Ser Lys Ser Leu Arg Pro Ser
       770                 775                 780
   Leu Asn Gly Arg Arg Ile Ser Asp Pro Gln Val Phe Ser Lys Glu Val
   785                 790                 795                 800
   Gln Thr Glu Ala Val Asp Asn Glu Pro Pro Asp Tyr Lys Ser Leu Ile
                   805                 810                 815
   Pro Leu Glu Arg Ala Val Ile Asn Gly Gln Leu Tyr Glu Glu Ser Glu
               820                 825                 830
   Asn Gln Asp Glu Asp Pro Asn Asp Glu Gly Ser Val Leu Ser Phe Lys
           835                 840                 845
   Cys Ser Gln Ser Thr Pro Cys Pro Val Asn Arg Lys Leu Trp Ile Pro
       850                 855                 860
   Trp Met Lys Ser Lys Glu Gly His Leu Gln Asn Gly Lys Met Gln Thr
   865                 870                 875                 880
   Lys Pro Asn Ala Asn Phe Val Gln Pro Gly Asp Leu Val Leu Ser His
                   885                 890                 895
   Thr Pro Gly Gln Pro Leu His Ile Lys Val Thr Pro Asp His Val Gln
               900                 905                 910
   Asn Thr Ala Thr Leu Glu Ile Thr Ser Pro Thr Thr Glu Ser Pro His
           915                 920                 925
   Ser Tyr Thr Ser Thr Ala Val Ile Pro Asn Cys Gly Thr Pro Lys Gln
       930                 935                 940
   Arg Ile Thr Ile Leu Gln Asn Ala Ser Ile Thr Pro Val Lys Ser Lys
   945                 950                 955                 960
   Thr Ser Thr Glu Asp Leu Met Asn Leu Glu Gln Gly Met Ser Pro Ile
                   965                 970                 975
   Thr Met Ala Thr Phe Ala Arg Ala Gln Thr Pro Glu Ser Cys Gly Ser
               980                 985                 990
   Leu Thr Pro Glu Arg Thr Met Ser  Pro Ile Gln Val Leu  Ala Val Thr
           995                 1000                1005
   Gly Ser Ala Ser Ser Pro Glu Gln Gly Arg Ser Pro Glu Pro Thr
   1010 1015 1020
   Glu Ile Ser Ala Lys His Ala Ile Phe Arg Val Ser Pro Asp Arg
   1025 1030 1035
   Gln Ser Ser Trp Gln Phe Gln Arg Ser Asn Ser Asn Ser Ser Ser
   1040 1045 1050
   Val Ile Thr Thr Glu Asp Asn Lys Ile His Ile His Leu Gly Ser
   1055 1060 1065
   Pro Tyr Met Gln Ala Val Ala Ser Pro Val Arg Pro Ala Ser Pro
   1070 1075 1080
   Ser Ala Pro Leu Gln Asp Asn Arg Thr Gln Gly Leu Ile Asn Gly
   1085 1090 1095
   Ala Leu Asn Lys Thr Thr Asn Lys Val Thr Ser Ser Ile Thr Ile
   1100 1105 1110
   Thr Pro Thr Ala Thr Pro Leu Pro Arg Gln Ser Gln Ile Thr Val
   1115 1120 1125
   Glu Pro Leu Leu Leu Pro His
   1130 1135
<210> 16
   <211> 1133
   <212> PRT
   <213> Homo sapiens
<400> 16
<210> 17
   <211> 1133
   <212> PRT
   <213> Homo sapiens
<400> 17
   Met Arg Ser Arg Gly Ser Asp Thr Glu Gly Ser Ala Gln Lys Lys Phe
   1 5 10 15
   Pro Arg His Thr Lys Gly His Ser Phe Gln Gly Pro Lys Asn Met Lys
   20 25 30
   His Arg Gln Gln Asp Lys Asp Ser Pro Ser Glu Ser Asp Val Ile Leu
   35 40 45
   Pro Cys Pro Lys Ala Glu Lys Pro His Ser Gly Asn Gly His Gln Ala
   50 55 60
   Glu Asp Leu Ser Arg Asp Asp Leu Leu Phe Leu Leu Ser Ile Leu Glu
   65 70 75 80
   Gly Glu Leu Gln Ala Arg Asp Glu Val Ile Gly Ile Leu Lys Ala Glu
   85 90 95
   Lys Met Asp Leu Ala Leu Leu Glu Ala Gln Tyr Gly Phe Val Thr Pro
   100 105 110
   Lys Lys Val Leu Glu Ala Leu Gln Arg Asp Ala Phe Gln Ala Lys Ser
   115 120 125
   Thr Pro Trp Gln Glu Asp Ile Tyr Glu Lys Pro Met Asn Glu Leu Asp
   130 135 140
   Lys Val Val Glu Lys His Lys Glu Ser Tyr Arg Arg Ile Leu Gly Gln
   145 150 155 160
   Leu Leu Val Ala Glu Lys Ser Arg Arg Gln Thr Ile Leu Glu Leu Glu
                   165                 170                 175
   Glu Glu Lys Arg Lys His Lys Glu Tyr Met Glu Lys Ser Asp Glu Phe
               180                 185                 190
   Ile Cys Leu Leu Glu Gln Glu Cys Glu Arg Leu Lys Lys Leu Ile Asp
           195                 200                 205
   Gln Glu Ile Lys Ser Gln Glu Glu Lys Glu Gln Glu Lys Glu Lys Arg
       210                 215                 220
   Val Thr Thr Leu Lys Glu Glu Leu Thr Lys Leu Lys Ser Phe Ala Leu
   225                 230                 235                 240
   Met Val Val Asp Glu Gln Gln Arg Leu Thr Ala Gln Leu Thr Leu Gln
                   245                 250                 255
   Arg Gln Lys Ile Gln Glu Leu Thr Thr Asn Ala Lys Glu Thr His Thr
               260                 265                 270
   Lys Leu Ala Leu Ala Glu Ala Arg Val Gln Glu Glu Glu Gln Lys Ala
           275                 280                 285
   Thr Arg Leu Glu Lys Glu Leu Gln Thr Gln Thr Thr Lys Phe His Gln
       290                 295                 300
   Asp Gln Asp Thr Ile Met Ala Lys Leu Thr Asn Glu Asp Ser Gln Asn
   305                 310                 315                 320
   Arg Gln Leu Gln Gln Lys Leu Ala Ala Leu Ser Arg Gln Ile Asp Glu
                   325                 330                 335
   Leu Glu Glu Thr Asn Arg Ser Leu Arg Lys Ala Glu Glu Glu Leu Gln
               340                 345                 350
   Asp Ile Lys Glu Lys Ile Ser Lys Gly Glu Tyr Gly Asn Ala Gly Ile
           355                 360                 365
   Met Ala Glu Val Glu Glu Leu Arg Lys Arg Val Leu Asp Met Glu Gly
       370                 375                 380
   Lys Asp Glu Glu Leu Ile Lys Met Glu Glu Gln Cys Arg Asp Leu Asn
   385                 390                 395                 400
   Lys Arg Leu Glu Arg Glu Thr Leu Gln Ser Lys Asp Phe Lys Leu Glu
   405 410 415
   Val Glu Lys Leu Ser Lys Arg Ile Met Ala Leu Glu Lys Leu Glu Asp
   420 425 430
   Ala Phe Asn Lys Ser Lys Gln Glu Cys Tyr Ser Leu Lys Cys Asn Leu
   435 440 445
   Glu Lys Glu Arg Met Thr Thr Lys Gln Leu Ser Gln Glu Leu Glu Ser
   450 455 460
   Leu Lys Val Arg Ile Lys Glu Leu Glu Ala Ile Glu Ser Arg Leu Glu
   465 470 475 480
   Lys Thr Glu Phe Thr Leu Lys Glu Asp Leu Thr Lys Leu Lys Thr Leu
   485 490 495
   Thr Val Met Phe Val Asp Glu Arg Lys Thr Met Ser Glu Lys Leu Lys
   500 505 510
   Lys Thr Glu Asp Lys Leu Gln Ala Ala Ser Ser Gln Leu Gln Val Glu
   515 520 525
   Gln Asn Lys Val Thr Thr Val Thr Glu Lys Leu Ile Glu Glu Thr Lys
   530 535 540
   Arg Ala Leu Lys Ser Lys Thr Asp Val Glu Glu Lys Met Tyr Ser Val
   545 550 555 560
   Thr Lys Glu Arg Asp Asp Leu Lys Asn Lys Leu Lys Ala Glu Glu Glu
   565 570 575
   Lys Gly Asn Asp Leu Leu Ser Arg Val Asn Met Leu Lys Asn Arg Leu
   580 585 590
   Gln Ser Leu Glu Ala Ile Glu Lys Asp Phe Leu Lys Asn Lys Leu Asn
   595 600 605
   Gln Asp Ser Gly Lys Ser Thr Thr Ala Leu His Gln Glu Asn Asn Lys
   610 615 620
   Ile Lys Glu Leu Ser Gln Glu Val Glu Arg Leu Lys Leu Lys Leu Lys
   625 630 635 640
   Asp Met Lys Ala Ile Glu Asp Asp Leu Met Lys Thr Glu Asp Glu Tyr
   645 650 655
   Glu Thr Leu Glu Arg Arg Tyr Ala Asn Glu Arg Asp Lys Ala Gln Phe
               660                 665                 670
   Leu Ser Lys Glu Leu Glu His Val Lys Met Glu Leu Ala Lys Tyr Lys
           675                 680                 685
   Leu Ala Glu Lys Thr Glu Thr Ser His Glu Gln Trp Leu Phe Lys Arg
       690                 695                 700
   Leu Gln Glu Glu Glu Ala Lys Ser Gly His Leu Ser Arg Glu Val Asp
   705                 710                 715                 720
   Ala Leu Lys Glu Lys Ile His Glu Tyr Met Ala Thr Glu Asp Leu Ile
                   725                 730                 735
   Cys His Leu Gln Gly Asp His Ser Val Leu Gln Lys Lys Leu Asn Gln
               740                 745                 750
   Gln Glu Asn Arg Asn Arg Asp Leu Gly Arg Glu Ile Glu Asn Leu Thr
           755                 760                 765
   Lys Glu Leu Glu Arg Tyr Arg His Phe Ser Lys Ser Leu Arg Pro Ser
       770                 775                 780
   Leu Asn Gly Arg Arg Ile Ser Asp Pro Gln Val Phe Ser Lys Glu Val
   785                 790                 795                 800
   Gln Thr Glu Ala Val Asp Asn Glu Pro Pro Asp Tyr Lys Ser Leu Ile
                   805                 810                 815
   Pro Leu Glu Arg Ala Val Ile Asn Gly Gln Leu Tyr Glu Glu Ser Glu
               820                 825                 830
   Asn Gln Asp Glu Asp Pro Asn Asp Glu Gly Ser Val Leu Ser Phe Lys
           835                 840                 845
   Cys Ser Gln Ser Thr Pro Cys Pro Val Asn Arg Lys Leu Trp Ile Pro
       850                 855                 860
   Trp Met Lys Ser Lys Glu Gly His Leu Gln Asn Gly Lys Met Gln Thr
   865                 870                 875                 880
   Lys Pro Asn Ala Asn Phe Val Gln Pro Gly Asp Leu Val Leu Ser His
                   885                 890                 895
   Thr Pro Gly Gln Pro Leu His Ile Lys Val Thr Pro Asp His Val Gln
               900                 905                 910
   Asn Thr Ala Thr Leu Glu Ile Thr Ser Pro Thr Thr Glu Ser Pro His
           915                 920                 925
   Ser Tyr Thr Ser Thr Ala Val Ile Pro Asn Cys Gly Thr Pro Lys Gln
       930                 935                 940
   Arg Ile Thr Ile Leu Gln Asn Ala Ser Ile Thr Pro Val Lys Ser Lys
   945                 950                 955                 960
   Thr Ser Thr Glu Asp Leu Met Asn Leu Glu Gln Gly Met Ser Pro Ile
                   965                 970                 975
   Thr Met Ala Thr Phe Ala Arg Ala Gln Thr Pro Glu Ser Cys Gly Ser
               980                 985                 990
   Leu Thr Pro Glu Arg Thr Met Ser  Pro Ile Gln Val Leu  Ala Val Thr
           995                 1000                1005
   Gly Ser  Ala Ser Ser Pro Glu  Gln Gly Arg Ser Pro  Glu Pro Thr
       1010                 1015                 1020
   Glu Ile  Ser Ala Lys His Ala  Ile Phe Arg Val Ser  Pro Asp Arg
       1025                 1030                 1035
   Gln Ser  Ser Trp Gln Phe Gln  Arg Ser Asn Ser Asn  Ser Ser Ser
       1040                 1045                 1050
   Val Ile  Thr Thr Glu Asp Asn  Lys Ile His Ile His  Leu Gly Ser
       1055                 1060                 1065
   Pro Tyr  Met Gln Ala Val Ala  Ser Pro Val Arg Pro  Ala Ser Pro
       1070                 1075                 1080
   Ser Ala  Pro Leu Gln Asp Asn  Arg Thr Gln Gly Leu  Ile Asn Gly
       1085                 1090                 1095
   Ala Leu  Asn Lys Thr Thr Asn  Lys Val Thr Ser Ser  Ile Thr Ile
       1100                 1105                 1110
   Thr Pro  Thr Ala Thr Pro Leu  Pro Arg Gln Ser Gln  Ile Thr Val
       1115                 1120                 1125
   Ser Asn  Ile Tyr Asn
       1130
<210> 18
   <211> 1135
   <212> PRT
   <213> Homo sapiens
<400> 18
<210> 19
   <211> 197
   <212> PRT
   <213> Homo sapiens
<400> 19
   Met Ala Ser Gln Lys Arg Pro Ser Gln Arg His Gly Ser Lys Tyr Leu
   1 5 10 15
   Ala Thr Ala Ser Thr Met Asp His Ala Arg His Gly Phe Leu Pro Arg
   20 25 30
   His Arg Asp Thr Gly Ile Leu Asp Ser Ile Gly Arg Phe Phe Gly Gly
   35 40 45
   Asp Arg Gly Ala Pro Lys Arg Gly Ser Gly Lys Val Pro Trp Leu Lys
   50 55 60
   Pro Gly Arg Ser Pro Leu Pro Ser His Ala Arg Ser Gln Pro Gly Leu
   65 70 75 80
   Cys Asn Met Tyr Lys Asp Ser His His Pro Ala Arg Thr Ala His Tyr
   85 90 95
   Gly Ser Leu Pro Gln Lys Ser His Gly Arg Thr Gln Asp Glu Asn Pro
   100 105 110
   Val Val His Phe Phe Lys Asn Ile Val Thr Pro Arg Thr Pro Pro Pro
   115 120 125
   Ser Gln Gly Lys Gly Arg Gly Leu Ser Leu Ser Arg Phe Ser Trp Gly
   130 135 140
   Ala Glu Gly Gln Arg Pro Gly Phe Gly Tyr Gly Gly Arg Ala Ser Asp
   145 150 155 160
   Tyr Lys Ser Ala His Lys Gly Phe Lys Gly Val Asp Ala Gln Gly Thr
   165 170 175
   Leu Ser Lys Ile Phe Lys Leu Gly Gly Arg Asp Ser Arg Ser Gly Ser
   180 185 190
   Pro Met Ala Arg Arg
   195
<210> 20
   <211> 186
   <212> PRT
   <213> Homo sapiens
<400> 20
   Met Ala Ser Gln Lys Arg Pro Ser Gln Arg His Gly Ser Lys Tyr Leu
   1 5 10 15
   Ala Thr Ala Ser Thr Met Asp His Ala Arg His Gly Phe Leu Pro Arg
   20 25 30
   His Arg Asp Thr Gly Ile Leu Asp Ser Ile Gly Arg Phe Phe Gly Gly
   35 40 45
   Asp Arg Gly Ala Pro Lys Arg Gly Ser Gly Lys Val Pro Trp Leu Lys
   50 55 60
   Pro Gly Arg Ser Pro Leu Pro Ser His Ala Arg Ser Gln Pro Gly Leu
   65 70 75 80
   Cys Asn Met Tyr Lys Asp Ser His His Pro Ala Arg Thr Ala His Tyr
                   85                  90                  95
   Gly Ser Leu Pro Gln Lys Ser His Gly Arg Thr Gln Asp Glu Asn Pro
               100                 105                 110
   Val Val His Phe Phe Lys Asn Ile Val Thr Pro Arg Thr Pro Pro Pro
           115                 120                 125
   Ser Gln Gly Lys Gly Ala Glu Gly Gln Arg Pro Gly Phe Gly Tyr Gly
       130                 135                 140
   Gly Arg Ala Ser Asp Tyr Lys Ser Ala His Lys Gly Phe Lys Gly Val
   145                 150                 155                 160
   Asp Ala Gln Gly Thr Leu Ser Lys Ile Phe Lys Leu Gly Gly Arg Asp
                   165                 170                 175
   Ser Arg Ser Gly Ser Pro Met Ala Arg Arg
               180                 185
<210> 21
   <211> 171
   <212> PRT
   <213> Homo sapiens
<400> 21
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Asp Ser His His Pro
       50                  55                  60
   Ala Arg Thr Ala His Tyr Gly Ser Leu Pro Gln Lys Ser His Gly Arg
   65                  70                  75                  80
   Thr Gln Asp Glu Asn Pro Val Val His Phe Phe Lys Asn Ile Val Thr
                   85                  90                  95
   Pro Arg Thr Pro Pro Pro Ser Gln Gly Lys Gly Arg Gly Leu Ser Leu
               100                 105                 110
   Ser Arg Phe Ser Trp Gly Ala Glu Gly Gln Arg Pro Gly Phe Gly Tyr
           115                 120                 125
   Gly Gly Arg Ala Ser Asp Tyr Lys Ser Ala His Lys Gly Phe Lys Gly
       130                 135                 140
   Val Asp Ala Gln Gly Thr Leu Ser Lys Ile Phe Lys Leu Gly Gly Arg
   145                 150                 155                 160
   Asp Ser Arg Ser Gly Ser Pro Met Ala Arg Arg
                   165                 170
<210> 22
   <211> 160
   <212> PRT
   <213> Homo sapiens
<400> 22
   Met Ala Ser Gln Lys Arg Pro Ser Gln Arg His Gly Ser Lys Tyr Leu
   1               5                   10                  15
   Ala Thr Ala Ser Thr Met Asp His Ala Arg His Gly Phe Leu Pro Arg
               20                  25                  30
   His Arg Asp Thr Gly Ile Leu Asp Ser Ile Gly Arg Phe Phe Gly Gly
           35                  40                  45
   Asp Arg Gly Ala Pro Lys Arg Gly Ser Gly Lys Asp Ser His His Pro
       50                  55                  60
   Ala Arg Thr Ala His Tyr Gly Ser Leu Pro Gln Lys Ser His Gly Arg
   65                  70                  75                  80
   Thr Gln Asp Glu Asn Pro Val Val His Phe Phe Lys Asn Ile Val Thr
                   85                  90                  95
   Pro Arg Thr Pro Pro Pro Ser Gln Gly Lys Gly Ala Glu Gly Gln Arg
               100                 105                 110
   Pro Gly Phe Gly Tyr Gly Gly Arg Ala Ser Asp Tyr Lys Ser Ala His
           115                 120                 125
   Lys Gly Phe Lys Gly Val Asp Ala Gln Gly Thr Leu Ser Lys Ile Phe
       130                 135                 140
   Lys Leu Gly Gly Arg Asp Ser Arg Ser Gly Ser Pro Met Ala Arg Arg
   145                 150                 155                 160
<210> 23
   <211> 253
   <212> PRT
   <213> Homo sapiens
<400> 23
<210> 24
   <211> 244
   <212> PRT
   <213> Homo sapiens
<400> 24
   Gly Leu Lys Gly Lys Arg Gly Asp Ser Gly Ser Pro Ala Thr Trp Thr
   1 5 10 15
   Thr Arg Gly Phe Val Phe Thr Arg His Ser Gln Thr Thr Ala Ile Pro
   20 25 30
   Ser Cys Pro Glu Gly Thr Val Pro Leu Tyr Ser Gly Phe Ser Phe Leu
   35 40 45
   Phe Val Gln Gly Asn Gln Arg Ala His Gly Gln Asp Leu Gly Thr Leu
   50 55 60
   Gly Ser Cys Leu Gln Arg Phe Thr Thr Met Pro Phe Leu Phe Cys Asn
   65 70 75 80
   Val Asn Asp Val Cys Asn Phe Ala Ser Arg Asn Asp Tyr Ser Tyr Trp
   85 90 95
   Leu Ser Thr Pro Ala Leu Met Pro Met Asn Met Ala Pro Ile Thr Gly
   100 105 110
   Arg Ala Leu Glu Pro Tyr Ile Ser Arg Cys Thr Val Cys Glu Gly Pro
   115 120 125
   Ala Ile Ala Ile Ala Val His Ser Gln Thr Thr Asp Ile Pro Pro Cys
   130 135 140
   Pro His Gly Trp Ile Ser Leu Trp Lys Gly Phe Ser Phe Ile Met Phe
   145 150 155 160
   Thr Ser Ala Gly Ser Glu Gly Thr Gly Gln Ala Leu Ala Ser Pro Gly
   165 170 175
   Ser Cys Leu Glu Glu Phe Arg Ala Ser Pro Phe Leu Glu Cys His Gly
   180 185 190
   Arg Gly Thr Cys Asn Tyr Tyr Ser Asn Ser Tyr Ser Phe Trp Leu Ala
           195                 200                 205
   Ser Leu Asn Pro Glu Arg Met Phe Arg Lys Pro Ile Pro Ser Thr Val
       210                 215                 220
   Lys Ala Gly Glu Leu Glu Lys Ile Ile Ser Arg Cys Gln Val Cys Met
   225                 230                 235                 240
   Lys Lys Arg His
<210> 25
   <211> 42
   <212> PRT
   <213> Homo sapiens
<400> 25